# EUROPÄISCHE PATENTSCHRIFT

(11) **EP 2 645 996 B1**
(45) Veröffentlichungstag und Bekanntmachung des Hinweises auf die Patenterteilung: **02.03.2016**
(21) Anmeldenummer: 11788458.5
(22) Anmeldetag: 28.11.2011
(51) Int. Cl.: A61K 9/16, A61K 9/20, A61K 9/28, A61K 9/50, A61K 31/44, A61K 31/4439, A61K 31/196

(54) **VERFAHREN ZUR HERSTELLUNG EINER PPI-HALTIGEN PHARMAZEUTISCHEN ZUBEREITUNG**
METHOD FOR PRODUCING A PHARMACEUTICAL COMPOSITION COMPRISING PROTON PUMP INHIBITORS
PROCÉDÉS POUR LA PREPARATION D'UNE COMPOSITION COMPRENNANT D'INHIBITEURS DE LA POMPE À PROTONS

(30) Priorität: 29.11.2010 DE 102010052847
(43) Veröffentlichungstag der Anmeldung: 09.10.2013
(73) Patentinhaber: Temmler Werke GmbH, 81673 München (DE)
(72) Erfinder: WEIß, Gerd, 81541 München (DE); PROFITLICH, Thomas, 81929 München (DE); SCHWITZER, Karl, 81929 München (DE); WAGNER, Cornelia, 81669 München (DE); SCHWITZER, Manfred, 81737 München (DE); HUBER, Bernhard, 81827 München (DE)
(74) Vertreter: Dannenberger, Oliver Andre
(86) Internationale Anmeldenummer: PCT/EP2011/071149
(87) Internationale Veröffentlichungsnummer: WO 2012/072570

(56) Entgegenhaltungen:
- EP-A2- 0 348 808
- EP-A2- 0 567 201
- WO-A2-2006/085335
- DE-A1- 19 626 045
- US-A1- 2002 054 913

## Beschreibung

Diese Erfindung betrifft ein Verfahren zur Herstellung einer pharmazeutischen Zubereitung, die einen Protonenpumpeninhibitor und gegebenenfalls ein nichtsteroidales Antirheumatikum in Form von Pellets enthält. Ferner betrifft die Erfindung durch das Verfahren erhältliche pharmazeutische Zubereitungen, insbesondere solche mit einem bestimmten Auflösungsprofil.

### Hintergrund des Gebiets

Protonenpumpen-Inhibitoren (PPI), auch als Protonenpumpenhemmer bezeichnet, sind Arzneistoffe, die die Bildung von Magensäure über die Hemmung der H⁺/K⁺-ATPase - einer sogenannten Protonenpumpe - in den Belegzellen des Magens unterdrücken. Omeprazol als bekanntester Arzneistoff aus der Gruppe von Protonenpumpen-Inhibitoren hat sich in der Therapie von Ulcus duodeni, Ulcus ventriculi, Refluxösophagitis und Zollinger-Ellision-Syndrom bewährt. Dabei kommen parenterale und feste perorale Arzneimittel zur Anwendung.

Omeprazol und dessen Derivate degradieren sehr schnell unter sauren Bedingungen zu unwirksamen Verbindungen, wobei offensichtlich schon der Kontakt des Wirkstoffes in festem Aggregationszustand mit sauren Gruppen (von z. B. magensaftresistenten Polymeren) zur Degradation führt. Feste perorale Arzneimittel (Tabletten, Pellets, Granulate) von Omeprazol und ähnlichen Wirkstoffen müssen deshalb vollständig gegen Magensaft geschützt werden. In wässriger Lösung hat Omeprazol beispielsweise, bei pH-Werten die unter 2 liegen, eine Halbwertszeit von weniger als 10 Minuten (Pilbrandt A. and Cederberg C. Scandinavian Journal of Gastroenterology, 1985, Suppl. 108).

Weiterhin ist zu berücksichtigen, dass die Resorption von Omeprazol im oberen Duodenum erfolgt, so dass eine möglichst rasche Freisetzung des Wirkstoffes nach Passage des Pylorus angestrebt werden muss.

Zwangsläufig ist daher Omeprazol mit einem Überzug zu versehen, der einerseits im sauren Milieu des Magens unlöslich ist, sich aber andererseits im neutralen bis schwach alkalischen Bereich des Duodenums auflöst.

Bei der Verwendung von beschichteten Pellets in pharmazeutischen Arzneimitteln ist jedoch zu beachten, dass diese eine Größe von deutlich unter 2000 µm aufweisen sollten, um eine Passage des Pylorus zu erzielen und um dadurch unregelmäßige Magenpassagezeiten zu vermeiden. Andererseits sollten die Pellets eine Mindestgröße von über 500 µm aufweisen, damit das Beschichten überhaupt noch wirtschaftlich effizient durchgeführt werden kann, da die Oberfläche im Verhältnis zum Gewicht überproportional ansteigt und somit bei gleicher Schichtdicke hohe Lackmengen benötigt werden (K. H. Bauer, et al., Coated Pharmaceutical Dosage Forms CRC Press, 1998, Seite 133 f.). Dabei ist neben dem bevorzugten Korngrößenbereich auch eine möglichst enge Korngrößenverteilung von Vorteil, um ein gleichmäßiges Befüllen zur Verabreichung von Pellets verwendeter Kapseln zu ermöglichen. Schließlich ist ein möglichst wirtschaftliches Verfahren mit schnellen Prozessabläufen und relativ hohen Ausbeuten von Vorteil.

Die europäische Patentanmeldung EP 0 247 983 A2 beschreibt die Herstellung pharmazeutischer Zubereitungen von Omeprazol, wobei der Wirkstoff zusammen mit einem alkalisch reagierenden Hilfsstoff zu Pelletkernen verarbeitet wird, die anschließend mit einer Isolierschicht und einer magensaftresistenten Schicht befilmt werden. Obwohl organische Lösungsmittel und Wasser als nachteilig für die Stabilität von Omeprazol genannt werden, erfolgt die Herstellung der Pelletkerne über eine wässrige Omeprazolsuspension (mit teilweise gelöstem Wirkstoff). Das Verfahren umfasst zahlreiche Herstellungsschritte und ist daher zeitaufwendig. Ferner erschwert der Einsatz von Wasser bei der Herstellung die Trocknung der erhaltenen Pellets.

Die Offenlegungsschrift DE 42 19 390 A1 beschreibt die Herstellung oraler Darreichungsformen wie Pellets oder Tabletten für Pantoprazol, die aus einem Kern, einer Zwischenschicht und einer magensaftresistenten äußeren Schicht bestehen. Die Herstellung der Pelletkerne erfolgt durch Beschichtung von Saccharose-Pellets mit Hydroxypropylmethylcellulose aus einer wässrigen Lösung und anschließendes Aufbringen des Wirkstoffs aus einer wässrigen alkoholischen Lösung in der Wirbelschicht. Dieses Herstellungsverfahren ist stark eingeschränkt, da es erfordert, dass bestimmte Füllstoffe bzw. Bindemittel nicht verwendet werden. Außerdem eignet sich dieses Verfahren nur bedingt für wasserempfindliche Wirkstoffe, da diese sich bei nicht genügender Trocknung zersetzen.

Die WO 03/080029 A1 offenbart die Herstellung pharmazeutischer Zubereitungen, die ein NO-abgebendes NSAID enthalten. Die NO-abgebenden NSAIDs werden bei der Herstellung von magensaftresistent befilmten Pellets in Wasser suspendiert und auf poröse Trägermaterialien aufgesprüht.

Die WO 97/25064 beschreibt die Herstellung oraler pharmazeutischer Zubereitungen, die neben einem magensaftresistent befilmten PPI auch einen oder mehrere NSAIDs enthalten können. Die Herstellung von Pelletkernen geschieht durch Besprühen der Pellets in der Wirbelschicht, wobei üblicherweise Wasser als Granulierflüssigkeit eingesetzt wird.

Die EP 0 567 201 offenbart Pellets, die hergestellt werden aus granulierten Mischungen von PPI mit mindestens einer basischen Komponente, mindestens einem pharmazeutisch annehmbaren Hilfsstoffes, und mindestens einem Alkohol, zum Erhalt von PPI-haltigen Kernen. Die Kerne werden getrocknet und anschliessend mit einer inerten Zwischenschicht überzogen und dann mit einer magensaftresistenten Schicht befilmt.

Es besteht daher ein Bedarf nach einem verbesserten Verfahren zur Herstellung pharmazeutischer Zubereitungen, die einen PPI (Protonenpumpeninhibitor) in Form von magensaftresistent beschichteten sphärischen Granulaten (Pellets) enthalten. Demzufolge ist die Aufgabe der Erfindung, ein Verfahren zur Herstellung von magensaftresistent beschichteten Pellets zur Verfügung zu stellen, mit dem bei kurzen Prozesszeiten höhere Ausbeuten der Pellets und in einem engen Korngrößenbereich erzielt werden können. Die Aufgabe wird gelöst durch Verwendung eines speziellen Agglomerationsverfahrens unter Einsatz eines Schnellmischers. Das Verfahren wird dabei insbesondere ohne Verwendung von Wasser oder wasserhaltigen Granulationsflüssigkeiten durch Wahl von Prozessparametern, die eine Rollbewegung des Granulats bevorzugen und hierdurch sphärische Agglomerate erzielen lassen, durchgeführt.

Das Verfahren ist insbesondere bei der Verwendung PPI-haltiger Pelletkerne von Vorteil, da durch den Ausschluss von Wasser bei der Herstellung die Degradationen des PPI weitest möglich vermieden werden kann.

Das Verfahren hat weiterhin den Vorteil, dass es weitestgehend unabhängig von den physiko-chemischen Eigenschaften der zu verarbeitenden Wirkstoffe insbesondere von der Wasserlöslichkeit ist. Hierdurch können auch mehrere Wirkstoffe gleichzeitig zu sphärischen Pelletkernen verarbeitet werden und in den anschließenden Prozessschritten somit gemeinsam behandelt werden.

Ein weiterer Vorteil des beschriebenen Verfahrens liegt in den kurzen Prozesszeiten und der damit verbundenen Wirtschaftlichkeit der Fertigung. Die Herstellung der Pelletkerne wird im Vergleich zu den üblichen Verfahren wie Beschichtung von Starterkernen im Pelletierteller bzw. in der Wirbelschicht und Extrusion / Sphäronisation wesentlich beschleunigt, da die Herstellung einer Wirkstoffsuspension entfällt, die Ausformung der sphärischen Granulate in kurzer Zeit erfolgt und deutlich kürzere Trocknungszeiten benötigt werden.

### Zusammenfassung der Erfindung

Die Erfindung betrifft ein Verfahren zur Herstellung einer pharmazeutischen Zubereitung, die einen PPI (Protonenpumpeninhibitor) in Form von sphärischen Granulaten (Pellets) enthält, umfassend die folgenden Schritte:
(a) Granulieren des PPI, mindestens einer basischen Komponente, mindestens eines pharmazeutisch annehmbaren Hilfsstoffes, und mindestens eines Alkohols, vorzugsweise von Ethanol und/oder Isopropanol, insbesondere von Isopropanol, unter Verwendung eines Schnellmischers, zum Erhalt von PPI-haltigen Kernen,
(b) Trocknen der Kerne,
(c) Überziehen der getrockneten Kerne mit einer inerten Zwischenschicht,
(d) Befilmen der überzogenen Kerne mit einer magensaftresistenten Schicht, und
(e) Trocknen der magensaftresistent befilmten Kerne auf eine Restfeuchte von kleiner oder gleich 1,5 Gew.-%, vorzugsweise kleiner oder gleich 1 Gew.-%, zum Erhalt von PPI-Pellets,
wobei die Rührflügelgeschwindigkeit des Schnellmischers größer oder gleich 50 U/min ist, vorzugsweise größer oder gleich 65 U/min ist, noch bevorzugter im Bereich von 70 bis 140 U/min liegt, und die Granulierzeit vorzugsweise kleiner als 20 Minuten, noch bevorzugter kleiner oder gleich 10 Minuten, besonders bevorzugt kleiner oder gleich 8 Minuten und am bevorzugtesten 5 bis 8 Minuten ist.

Das Granulieren in Schritt (a) geschieht dabei ohne Verwendung von Wasser oder von wasserhaltigen Lösungsmitteln. Es ist insbesondere bevorzugt, dass das Granulieren in Abwesenheit von Wasser ausgeführt wird, soweit dies technisch möglich ist. Abwesenheit von Wasser bedeutet in diesem Zusammenhang, dass das Lösungsmittel vorzugsweise nicht mehr als 10,0 Gew.-%, insbesondere nicht mehr als 5,0 Gew.-%, bevorzugt nicht mehr als 1,0 Gew.-% Wasser enthält. Es ist insbesondere bevorzugt 96 Vol.-% Ethanol bzw. 99,9 Gew.-% Ethanol oder 99,5 Gew.-% Isopropanol zu verwenden. Es kann dabei auch von Vorteil sein, Mischungen von Lösungsmitteln zu verwenden.

Ferner betrifft die Erfindung ein Verfahren zur Herstellung einer pharmazeutischen Zubereitung, die neben dem PPI auch ein NSAID (nichtsteroidales Antirheumatikum) in Form von Pellets enthält, wobei ein Teil des NSAID
(i) in Form eines Salzes, beispielsweise als Natrium-Salz, zusammen mit dem PPI gemäß Schritten (a) bis (e) granuliert, getrocknet, überzogen, befilmt und erneut getrocknet wird und/oder
(ii) in freier Form oder in Form eines Salzes, beispielsweise als Natrium-Salz, getrennt von dem PPI zusammen mit mindestens einem pharmazeutisch annehmbaren Hilfsstoff und mindestens einem Alkohol, vorzugsweise von Ethanol und/oder Isopropanol, insbesondere von Isopropanol, unter Verwendung eines Schnellmischers, zum Erhalt von NSAID-haltigen Kernen granuliert, die Kerne getrocknet, mit einer
magensaftresistenten Schicht befilmt und die magensaftresistent befilmten Kerne auf eine Restfeuchte von kleiner oder gleich 4,0 Gew.-%, vorzugsweise von kleiner oder gleich 3,0 Gew.-%, besonders bevorzugt von kleiner oder gleich 2,5 Gew.-% getrocknet werden, zum Erhalt von magensaftresistent befilmten NSAID-Pellets, und Mischen der magensaftresistent befilmten NSAID-Pellets mit den (gegebenenfalls NSAID-haltigen) PPI-Pellets, wobei die Rührflügelgeschwindigkeit des Schnellmischers größer oder gleich 50 U/min ist, vorzugsweise größer oder gleich 65 U/min, noch bevorzugter im Bereich von 70 bis 140 U/min liegt, und die Granulierzeit vorzugsweise kleiner als 20 Minuten, noch bevorzugter kleiner oder gleich 10 Minuten, besonders bevorzugt kleiner oder gleich 8 Minuten und am bevorzugtesten 5 bis 8 Minuten ist.

Das Granulieren des NSAID sowohl in Variante (i) als auch in Variante (ii) geschieht dabei vorzugsweise ohne Verwendung von Wasser oder von wasserhaltigen Lösungsmitteln. Es ist insbesondere bevorzugt, dass das Granulieren in Abwesenheit von Wasser ausgeführt wird, soweit dies technisch möglich ist. Abwesenheit von Wasser bedeutet in diesem Zusammenhang, dass das Lösungsmittel vorzugsweise nicht mehr als 10,0 Gew.-%, insbesondere nicht mehr als 5,0 Gew.-%, bevorzugt nicht mehr als 1,0 Gew.-% Wasser enthält. Es ist insbesondere bevorzugt 96 Vol.-% Ethanol bzw. 99,9 Gew.-% Ethanol oder 99,5 Gew.-% Isopropanol zu verwenden. Es kann dabei auch von Vorteil sein, Mischungen von Lösungsmitteln zu verwenden.

Weiterhin betrifft die Erfindung das obige Verfahren zur Herstellung einer pharmazeutischen Zubereitung die neben dem PPI auch ein NSAID in Form von Pellets enthält, wobei ein (weiterer) Teil des NSAID zu Pellets verarbeitet und diese mit einer für das NSAID retardiert durchlässigen Diffusionsmembran umhüllt werden, zum Erhalt retardiert freisetzender NSAID-Pellets, und Vermischen der retardiert freisetzenden NSAID-Pellets mit den PPI-Pellets und gegebenenfalls den magensaftresistenten NSAID-Pellets. Bei der Herstellung retardiert freisetzender NSAID-Pellets ist es bevorzugt, dass diese auf eine Restfeuchte von kleiner oder gleich 2 Gew.-%, vorzugsweise von kleiner oder gleich 1,5 Gew.-%, besonders bevorzugt von kleiner oder gleich 1,0 Gew.-% getrocknet werden.

Des Weiteren betrifft die Erfindung pharmazeutische Zubereitungen, die durch diese Herstellungsverfahren erhältlich sind, insbesondere solche bei denen die Pellets in einer Gelatinekapsel, vorzugsweise einer Hartgelatinekapsel eingeschlossen sind. Bevorzugt sind ebenfalls derartige pharmazeutische Zubereitungen mit einer Auflösungsgeschwindigkeit, die beim Messen *in vitro* in einer Drehkörbchen- bzw. Blattrührerapparatur nach dem Europäischen Arzneibuch Version 6 in Phosphatpuffer bei einer Lösungsmittelmenge von 900 ml bei 100 U/min und 37 °C im wesentlichen dem folgenden Auflösungsmuster entspricht:
(i) nach 2 h Messzeit bei pH 1,2 sind 10 % oder weniger, vorzugsweise 5 % oder weniger des gesamten PPI gelöst, und
(ii) nach 2 h Messzeit bei pH 1,2 und nach 10 min Messzeit bei pH 6,8 sind 80 % oder mehr, vorzugsweise 90 % oder mehr des gesamten PPI gelöst.

### Kurze Beschreibung der Figuren

**Figur 1** zeigt zwei Freisetzungskurven von erfindungsgemäßen Omeprazol-haltigen Pelletkernen (unlackiert) bei einem pH von 6,8.
**Figur 2** zeigt die Freisetzungskurven der erfindungsgemäßen Omeprazol-haltigen Pelletkerne bei pH 6,8 nach Einlagerung unter verschiedenen Stressbedingungen.
**Figur 3a** und **Figur 3b** zeigen die Freisetzungskurven der erfindungsgemäßen magensaftresistent befilmten Omeprazol-haltigen Kerne bei pH 6,8 nach 2 Stunden Vorbehandlung bei pH 1,2 bzw. pH 4,5.
**Figur 4** zeigt das in vitro Freisetzungsprofil erfindungsgemäßer magensaftresistenter Diclofenac-haltiger Pellets bei pH 1,2 (120 min), gefolgt durch pH 6,8.
**Figur 5** zeigt das in vitro Freisetzungsprofil erfindungsgemäßer verzögert freisetzender Diclofenac-haltiger Pellets bei pH 1,2 (120 min), gefolgt durch pH 6,8.
**Figur 6** zeigt das in vitro Freisetzungsprofil einer erfindungsgemäßen Diclofenac-haltigen Pelletmischung bei pH 1,2 (120 min), gefolgt durch pH 6,8.
**Figur 7** zeigt das in vitro Freisetzüngsprofil einer erfindungsgemäßen Omeprazol-haltigen und Diclofenac-haltigen Pelletmischung bei pH 1,2 (120 min) und anschließend bei pH 6,8.
**Figur 8** zeigt das in vitro Freisetzungsprofil einer erfindungsgemäßen Kombinationszubereitung von magensaftresistent befilmten Pellets umfassend 20 mg Omeprazol und 25 mg Diclofenac bei pH 1,2 (120 min) und anschließend bei pH 6,8.
**Figur 9** zeigt das in vitro Freisetzungsprofil einer erfindungsgemäßen Kombinationszubereitung von magensaftresistent befilmten Pellets umfassend 10 mg Omeprazol und 25 mg Diclofenac bei pH 1,2 (120 min) und anschließend bei pH 6,8.

### Detaillierte Beschreibung der Erfindung

Die Erfindung betrifft ein Verfahren zur Herstellung einer pharmazeutischen Zubereitung, die einen PPI (Protonenpumpeninhibitor) in Form von sphärischen Granulaten (Pellets) enthält, umfassend die folgenden Schritte:
(a) Granulieren des PPI, mindestens einer basischen Komponente, mindestens eines pharmazeutisch annehmbaren Hilfsstoffes, und mindestens eines Alkohols, vorzugsweise von Ethanol und/oder Isopropanol, insbesondere von Isopropanol, unter Verwendung eines Schnellmischers, zum Erhalt von PPI-haltigen Kernen,
(b) Trocknen der Kerne,
(c) Überziehen der getrockneten Kerne mit einer inerten Zwischenschicht,
(d) Befilmen der überzogenen Kerne mit einer magensaftresistenten Schicht, und
(e) Trocknen der magensaftresistent befilmten Kerne auf eine Restfeuchte von kleiner oder gleich 1,5 Gew.-%, vorzugsweise kleiner oder gleich 1 Gew.-%, zum Erhalt von PPI-Pellets,
wobei die Rührflügelgeschwindigkeit des Schnellmischers größer oder gleich 50 U/min ist, vorzugsweise größer oder gleich 65 U/min ist, noch bevorzugter im Bereich von 70 bis 140 U/min liegt, und die Granulierzeit vorzugsweise kleiner als 20 Minuten, noch bevorzugter kleiner oder gleich 10 Minuten, besonders bevorzugt kleiner oder gleich 8 Minuten und am bevorzugtesten 5 bis 8 Minuten ist. Das Granulieren in Schritt (a) geschieht in Abwesenheit von Wasser, wie oben definiert.

In diesem Verfahren ist es besonders bevorzugt, wenn in Schritt (a) Magnesiumcarbonat als basische Komponente und Isopropanol als Alkohol eingesetzt wird, insbesondere bei einer Rührflügelgeschwindigkeit des Schnellmischers im Bereich von 70 bis 140 U/min liegt, da dann bei einer Granulierzeit von kleiner oder gleich 10 Minuten (noch besser von kleiner oder gleich 8 Minuten und am besten 5 bis 8 Minuten) PPI-haltige Pellets erhalten werden können, die sich durch eine enge Korngrößenverteilung und eine hohe Ausbeute an sphärischen Pellets im Bereich von 700 *µ*m bis 1250 *µ*m auszeichnen.

Bei dem PPI handelt es sich dabei vorzugsweise um Omeprazol, Esomeprazol, Lansoprazol, Pantoprazol oder Rabeprazol, insbesondere um Omeprazol.

In einer weiteren Ausführungsform dient das erfindungsgemäße Verfahren zur Herstellung einer pharmazeutischen Zubereitung die neben dem PPI weiterhin ein NSAID (nichtsteroidales Antirheumatikum) in Form von Pellets enthält, wobei ein Teil des NSAID in Form eines Salzes, beispielsweise als Natrium-Salz oder als Kalium-Salz, zusammen mit dem PPI gemäß Schritten (a) bis (e) granuliert, getrocknet, überzogen, befilmt und erneut getrocknet wird, so dass PPI-Pellets erhalten werden, bei denen die Kerne sowohl ein PPI als auch ein NSAID enthalten.

Alternativ kann die pharmazeutischen Zubereitung die neben dem PPI noch ein NSAID in Form von Pellets enthält hergestellt werden, in dem das NSAID in freier Form oder in Form eines Salzes, beispielsweise als Natrium-Salz oder als Kalium-Salz, getrennt von dem PPI zusammen mit mindestens einem pharmazeutisch annehmbaren Hilfsstoff und mindestens einem Alkohol, vorzugsweise von Ethanol und/oder Isopropanol, insbesondere von Isopropanol, vorzugsweise in Abwesenheit von Wasser, wie oben definiert (z.B. unter Verwendung von 96 Vol.-% Ethanol oder 99,9 Gew.-% Ethanol bzw. 99,5 Gew.-% Isopropanol), unter Verwendung eines Schnellmischers, zum Erhalt von NSAID-haltigen Kernen granuliert, die Kerne getrocknet, mit einer magensaftresistenten Schicht befilmt und die magensaftresistent befilmten Kerne auf eine Restfeuchte von kleiner oder gleich 4,0 Gew.-%, vorzugsweise von kleiner oder gleich 3,0 Gew.-%, besonders bevorzugt von kleiner oder gleich 2,5 Gew.-% getrocknet werden, zum Erhalt von magensaftresistent befilmten NSAID-Pellets, und Mischen der magensaftresistent befilmten NSAID-Pellets mit den (gegebenenfalls NSAID-haltigen) PPI-Pellets, wobei die Rührflügelgeschwindigkeit des Schnellmischers größer oder gleich 50 U/min ist, vorzugsweise größer oder gleich 65 U/min, noch bevorzugter im Bereich von 70 bis 140 U/min liegt, und die Granulierzeit vorzugsweise kleiner als 20 Minuten, noch bevorzugter kleiner oder gleich 10 Minuten, besonders bevorzugt kleiner oder gleich 8 Minuten und am bevorzugtesten 5 bis 8 Minuten ist.

Das erfindungsgemäße Verfahren hat den Vorteil, dass es die Herstellung von pharmazeutischen Zubereitungen ermöglicht, welche die gleichzeitige Verabreichung sowohl von PPIs, wie z.B. von Omeprazol, als auch von NSAIDs, wie z.B. Diclofenac erlaubt. Dabei liegt das NSAID vorzugsweise zum einen Teil in magensaft-resistent beschichteten Pellets, zum anderen Teil in retardiert freisetzenden NSAID-Pellets vor. Bei dem Verfahren zur Herstellung dieser pharmazeutischen Zubereitungen wird ein Teil des NSAID zusammen mit mindestens einem pharmazeutisch annehmbaren Hilfsstoff zu NSAID-haltigen Kernen verarbeitet und diese mit einer für das NSAID retardiert durchlässigen Diffusionsmembran umhüllt, zum Erhalt von retardiert freisetzenden NSAID-Pellets.

Gegebenenfalls werden die retardiert freisetzenden NSAID-Pellets auf eine Restfeuchte von kleiner oder gleich 2 Gew.-%, vorzugsweise von kleiner oder gleich 1,5 Gew.-%, besonders bevorzugt von kleiner oder gleich 1,0 Gew.-% getrocknet. Die gegebenenfalls getrockneten retardiert freisetzenden NSAID-Pellets werden anschließend mit den PPI-Pellets und gegebenenfalls den magensaftresistenten NSAID-Pellets vermischt.

Vorzugsweise wird das NSAID ausgewählt aus Diclofenac, Ibuprofen, Ketoprofen, Naproxen, Indometacin, Piroxicam, Meloxicam, Acetylsalicylsäure, Celecoxib, Parecoxib und Etoricoxib. Im Rahmen der vorliegenden Erfindung ist es besonders bevorzugt Diclofenac, insbesondere Diclofenac-Natrium, als NSAID zu verwenden.

Bei dem erfindungsgemäßen Verfahren beträgt das Stoffmengenverhältnis von NSAID in magensaftresistenten Pellets zu NSAID in retardiert freisetzenden Pellets 0,1:1 bis 1:1, vorzugsweise 0,5:1 bis 1:1, insbesondere 0,5:1.

Gemäß dem erfindungsgemäßen Verfahren ist es besonders bevorzugt, dass die PPI-Pellets nach der Herstellung einen Wassergehalt von kleiner oder gleich 1,0 Gew.-%, vorzugsweise von kleiner oder gleich 0,5 Gew.-% aufweisen.

Bei der mindestens einen basischen Komponente, die im erfindungsgemäßen Verfahren verwendet wird, handelt es sich insbesondere um Magnesiumcarbonat, Magnesiumoxid, Magnesiumhydroxid, Aluminiumcarbonat, Aluminiumhydroxid, Calciumcarbonat, Calciumphosphat, Calciumcitrat, Natriumcarbonat, Natriumhydrogencarbonat, Natriumphosphat, Natriumcitrat, Kaliumcarbonat, Kaliumphosphat und/oder Kaliumcitrat, vorzugsweise um Magnesiumcarbonat.

Bei dem im erfindungsgemäßen Verfahren verwendeten mindestens einen Hilfsstoff handelt es sich vorzugsweise um Mannitol, Sorbitol, Isomalt, hochdisperses Siliziumdioxid, Mikrokristalline Cellulose, Dextrin, Maltodextrin, Maisstärke, Saccharose, Lactose und/oder Natriumlaurylsulfat, vorzugsweise um Mannitol und/oder Natriumlaurylsulfat.

Die oben aufgeführte inerte Zwischenschicht umfasst dabei insbesondere Hydroxypropylcellulose, Hydroxypropylmethylcellulose, Polyvinylpyrrolidon, Polyvinylalkohol und Mischungen davon, insbesondere Hydroxypropylmethylcellulose. Die inerte Zwischenschicht kann ferner Antiklebemittel wie Talkum enthalten.

Die magensaftresistente Schicht wird vorzugsweise unabhängig ausgewählt aus: Methacrylsäure-Ethylacrylat-Copolymer, Methacrylsäure-Methylmethacrylat-Copolymer, Polyvinylacetatphthalat, Hydroxypropylmethylcelluloseacetatphthalat, Celluloseacetatphthalat, und Hydroxypropylmethylcelluloseacetatsuccinat, insbesondere Methacrylsäure-Ethylacrylat-Copolymer, wie z.B. Eudragit L 30 D-55®. Die magensaftresistente Schicht kann ferner Weichmacher wie Polyethylenglykol (z.B. PEG 6000) oder Triethylcitrat, Antiklebemittel wie Talkum und/oder Weisspigment wie Titandioxid enthalten.

Bei den im erfindungsgemäßen Verfahren verwendeten Schnellmischern handelt es sich vorzugsweise um solche, die erhältlich sind von DIOSNA Dierks & Söhne GmbH, Deutschland (z.B. Diosna P1200 bzw. VAC 1200), Glatt Prozess Technology GmbH, Deutschland (z.B. Glatt VG 1500 bzw. TDG1500), L.B. Bohle Maschinen+Verfahren GmbH, Deutschland, Collette / GEA Pharma Systems AG, Schweiz (z.B. Collette Gral 1200), Zanchetta / I.M.A. Industria Macchine Automatiche S.p.A., Italien, Fielder-Aeromatic / GEA Pharma Systems AG, Schweiz sowie Gebr. Lödige Maschinenbau GmbH, Deutschland.

Die vorliegende Erfindung betrifft in einer weiteren Ausführungsform eine pharmazeutische Zubereitung erhältlich durch ein erfindungsgemäßes Verfahren, insbesondere eine Zubereitung die Omeprazol als PPI und Diclofenac, insbesondere Diclofenac-Natrium als NSAID enthält. Dabei ist es besonders bevorzugt, wenn das Diclofenac sowohl in Form magensaftresistent beschichteter Pellets als aus in Form von Pellets mit verzögerter Freisetzung vorliegt. Dabei ist es besonders vorteilhaft, wenn das Gewichts-Verhältnis Omeprazol : Diclofenac-Natrium (magensaftresistent umhüllt) : Diclofenac-Natrium (verzögerte Freisetzung) 1,0 : 1,0 - 3,0 : 1,5 - 5,0, insbesondere 1,0 : 1,25 - 2,5 : 2,5 - 5,0, vorzugsweise 1,0 : 1,25 : 2,5 oder 1,0 : 2,5 : 5,0, besonders bevorzugt 1,0 : 1,25 : 2,5 beträgt.

In einer besonders vorteilhaften Ausführungsform stellt die vorliegende Erfindung eine pharmazeutische Zubereitung bereit, erhältlich mit dem erfindungsgemäßen Verfahren, wobei die Pellets eine Freisetzungsgeschwindigkeit besitzen, die beim Messen *in vitro* in einer Drehkörbchen- bzw. Blattrührerapparatur nach dem Europäischen Arzneibuch Version 6 in Phosphatpuffer bei einer Lösungsmittelmenge von 900 ml (oder 1000 ml) bei 100 U/min und 37 °C im wesentlichen dem folgenden Auflösungsmuster entspricht: nach 10 min Messzeit werden bei pH 6,8 80% oder mehr, vorzugsweise 90% oder mehr des PPI freigesetzt.

In einer weiteren besonders vorteilhaften Ausführungsform stellt die vorliegende Erfindung eine pharmazeutische Zubereitung bereit, erhältlich mit dem erfindungsgemäßen Verfahren, wobei die Pellets eine Auflösungsgeschwindigkeit besitzen, die beim Messen *in vitro* in einer Drehkörbchen- bzw. Blattrührerapparatur nach dem Europäischen Arzneibuch Version 6 in Salzsäure bzw. Phosphatpuffer bei einer Lösungsmittelmenge von 900 ml bei 100 U/min und 37 °C im wesentlichen dem folgenden Auflösungsmuster entspricht: (i) nach 2 h Messzeit bei pH 1,2 sind 10 % oder weniger, vorzugsweise 5 % oder weniger des gesamten PPI gelöst, und (ii) nach 2 h Messzeit bei pH 1,2 und nach 10 min Messzeit bei pH 6,8 sind 80% oder mehr, vorzugsweise 90 % oder mehr des gesamten PPI gelöst.

In einer weiteren besonders vorteilhaften Ausführungsform stellt die vorliegende Erfindung eine pharmazeutische Zubereitung bereit, erhältlich mit dem erfindungsgemäßen Verfahren, wobei die magensaftresistent befilmten Pellets eine Auflösungsgeschwindigkeit besitzen, die beim Messen *in vitro* in einer Drehkörbchen- bzw. Blattrührerapparatur nach dem Europäischen Arzneibuch Version 6 in Salzsäure bzw. Phosphatpuffer bei einer Lösungsmittelmenge von 900 ml bei 100 U/min und 37 °C im wesentlichen dem folgenden Auflösungsmuster entspricht: (i) nach 2 h Messzeit bei pH 1,2 sind 10 % oder weniger, vorzugsweise 5 % oder weniger des NSAID gelöst, und (ii) nach 1 h Messzeit (im Anschluss an die zweistündige Messung bei pH 1,2 und/oder als eigenständige Messung) bei pH 6,8 sind 80 % oder mehr des NSAID gelöst.

In einer weiteren besonders vorteilhaften Ausführungsform stellt die vorliegende Erfindung eine pharmazeutische Zubereitung bereit, erhältlich mit dem erfindungsgemäßen Verfahren, die beim Messen *in vitro* in einer Drehkörbchen- bzw. Blattrührerapparatur nach dem Europäischen Arzneibuch Version 6 in Salzsäure bzw. Phosphatpuffer bei einer Lösungsmittelmenge von 900 ml bei 100 U/min und 37 °C die im wesentlichen folgende Auflösungsgeschwindigkeit aufweist:
(i) nach 2 h Messzeit bei pH 1,2 sind 10 % oder weniger, vorzugsweise 5 % oder weniger des gesamten PPI und 10 % oder weniger, vorzugsweise 5 % oder weniger des gesamten NSAID gelöst,
(ii) nach 10 min Messzeit bei pH 6,8 sind 80 % oder mehr, vorzugsweise 90 % oder mehr des gesamten PPI gelöst, und
(iii) nach 1 h Messzeit bei pH 6,8 sind 40 bis 70 % des gesamten NSAID gelöst, und über einen Zeitraum von weiteren 5 h Messzeit bei pH 6,8 werden weitere 2,5 bis 10 % des gesamten NSAID pro h gelöst.

Die vorstehend beschriebenen Messungen (ii) und (iii) bei einem pH von 6,8 können im Anschluss an die Messung (i) bei pH 1,2 ausgeführt werden kann, aber auch ohne vorherige Messung (i) bei einem pH von 1,2. Vorzugsweise zeigt die erfindungsgemäße Zusammensetzung, die sowohl PPI-Pellets als auch NSAID-Pellets umfasst:
(a) eine sehr geringe Freisetzung (d.h. 10 % oder weniger, vorzugsweise 5 % oder weniger) der jeweiligen Wirkstoffe innerhalb von 120 min bei pH 1,2,
(b) eine sehr schnelle Freisetzung des PPI (größer 80 %, vorzugsweise größer 90%) über einen sehr kurzen Zeitraum (in 20 min oder weniger, insbesondere in 10 min oder weniger) bei pH 6,8,
(c) eine sehr schnelle Freisetzung eines Teils des NSAID (etwa 40 bis 70 %, insbesondere etwa 50 bis 60 %) über einen kurzen Zeitraum (40 bis 80 min, insbesondere etwa 40 bis 60 min) bei pH 6,8, und eine verzögerte Freisetzung eines Teils des NSAID (etwa 20 bis 60 %, insbesondere etwa 30 bis 50 %) nach der schnellen Freisetzung über einen längeren Zeitraum (etwa 5 h oder mehr, vorzugsweise etwa 7 h oder mehr, besonders bevorzugt etwa 12 h oder mehr), wobei insbesondere eine Auflösungsgeschwindigkeit des NSAID von 2,5 %/h bis 10 %/h, vorzugsweise von 4 %/h bis 8 %/h über diesen längeren Zeitraum vorteilhaft ist.

Weiter beschrieben wird eine pharmazeutische Zubereitung, die PPI-haltige und NSAID-haltige Pellets umfasst, wobei:
die PPI-haltigen Pellets einen Kern umfassen, der den PPI, mindestens einer basischen Komponente und mindestens einen pharmazeutisch annehmbaren Hilfsstoff, umfasst, wobei der Kern mit einer inerten Zwischenschicht überzogen und mit einer magensaftresistenten Schicht befilmt ist, und
ein Teil der NSAID-haltigen Pellets einen Kern umfassen, der den NSAID und mindestens einen pharmazeutisch annehmbaren Hilfsstoff, umfasst, wobei der Kern mit einer magensaftresistenten Schicht befilmt ist, und
ein weiterer Teil der NSAID-haltigen Pellets einen Kern umfassen, der den NSAID und mindestens einen pharmazeutisch annehmbaren Hilfsstoff, umfasst, wobei der Kern mit einer für das NSAID retardiert durchlässigen Diffusionsmembran umhüllt ist,
wobei:
   das NASID in freier Form oder in Form eines Salzes, beispielsweise als Natrium-Salz, vorliegt,
   die pharmazeutische Zubereitung 5 mg bis 40 mg, vorzugsweise 10 mg bis 30 mg, insbesondere 10 mg, 15 mg, 20 mg oder 30 mg PPI, und 50 mg bis 150 mg, vorzugsweise 50 mg, 75 mg oder 150 mg, insbesondere 75 mg NSAID, enthält,
   der PPI ausgewählt ist aus Omeprazol, Esomeprazol, Lansoprazol, Pantoprazol und Rabeprazol, vorzugsweise aus Omeprazol,
   das NSAID ausgewählt ist aus Diclofenac, Ibuprofen, Ketoprofen, Naproxen, Indometacin, Piroxicam, Meloxicam, Acetylsalicylsäure, Celecoxib, Parecoxib und Etoricoxib, vorzugsweise aus Diclofenac,
   und die mindestens eine basische Komponente unabhängig ausgewählt ist aus der Gruppe bestehend aus: Magnesiumcarbonat, Magnesiumoxid, Magnesiumhydroxid, Aluminiumcarbonat, Aluminiumhydroxid, Calciumcarbonat, Calciumphosphat, Calciumcitrat, Natriumcarbonat, Natriumhydrogencarbonat, Natriumphosphat, Natriumcitrat, Kaliumcarbonat, Kaliumphosphat und Kaliumcitrat, und vorzugsweise Magnesiumcarbonat ist.

Außerdem beschrieben wird eine pharmazeutische Zubereitung, die PPI-haltige und NSAID-haltige Pellets umfasst, wobei:
die PPI-haltigen Pellets einen Kern umfassen, der den PPI, mindestens einer basischen Komponente und mindestens einen pharmazeutisch annehmbaren Hilfsstoff, umfasst, wobei der Kern mit einer inerten Zwischenschicht überzogen und mit einer magensaftresistenten Schicht befilmt ist, und
ein Teil der NSAID-haltigen Pellets einen Kern umfassen, der den NSAID und mindestens einen pharmazeutisch annehmbaren Hilfsstoff, umfasst, wobei der Kern mit einer magensaftresistenten Schicht befilmt ist, und
ein weiterer Teil der NSAID-haltigen Pellets einen Kern umfassen, der den NSAID und mindestens einen pharmazeutisch annehmbaren Hilfsstoff, umfasst, wobei der Kern mit einer für das NSAID retardiert durchlässigen Diffusionsmembran umhüllt ist,
wobei:
   die pharmazeutische Zubereitung 5 mg bis 40 mg, vorzugsweise 10 mg bis 30 mg, insbesondere 10 mg, 15 mg oder 20 mg PPI, und 50 mg bis 150 mg, vorzugsweise 50 mg, 75 mg oder 150 mg, insbesondere 75 mg NSAID, enthält,
   der PPI als Omeprazol und das NASID als Diclofenac oder Diclofenac-Natrium vorliegt,
   und die mindestens eine basische Komponente Magnesiumcarbonat ist.

Die erfindungsgemäßen pharmazeutischen Zubereitungen, die sowohl PPI als auch NSAID umfassen, wobei der PPI schnell freisetzend ist und das NSAID teilweise schnell freisetzend und teilweise verzögernd freisetzend ist, weisen den Vorteil auf, dass (i) ein schneller Wirkungseintritt des NSAID erfolgt, (ii) der erforderliche Blutspiegel des NSAID über einen Zeitraum von mehreren Stunden aufrechterhalten wird und (iii) Nebenwirkungen des NSAID, die insbesondere bei Langzeitanwendung auftreten können, wie Magen- und Darmbeschwerden, die durch eine Hemmung der in der Magenschleimhaut vorkommenden COX-1 hervorgerufen werden können, durch die gleichzeitige Gabe eines PPI reduziert oder sogar ganz vermieden werden. Außerdem wird die Compliance gefördert, da nicht zwei oder gar drei unterschiedliche Medikamente eingenommen werden müssen. Dadurch wird zusätzlich die Anwendungssicherheit erhöht.

Die folgenden Beispiele veranschaulichen die Herstellung und Charakterisierung des erfindungsgemäßen Verfahrens sowie dessen Verwendung zur Herstellung der pharmazeutischen Zubereitungen. Wenngleich diese Beispiele spezielle Ausführungsformen der Erfindung beschreiben, dienen diese nur zur Veranschaulichung der Erfindung und sollen nicht als die Erfindung in irgendeiner Weise einschränkend aufgefasst werden. Wie der Fachmann weiß, können zahlreiche Änderungen daran durchgeführt werden, ohne vom Schutzumfang der Erfindung, wie er durch die beigefügten Patentansprüche definiert wird, abzuweichen.

### Beispiele

Es sei darauf hingewiesen, dass sich die %-Angaben, wenn nicht anders angegeben, auf Gewichtsprozent (Gew.-%) beziehen.

Die Restfeuchte (Wassergehalt) der magensaftresistent oder retardierend befilmten Kerne (PPI-Pellets bzw. NSAID-Pellets) nach dem Trocknen wurde mittels Karl Fischer Titration gemäß dem Europäischen Arzneibuch, Version 6, (z.B. am Gerät Methrom KF Titrino 701) unter Verwendung von Hydranal-Reagenzien bestimmt.

Der Trocknungsverlust wurde mit einem Halogen-Feuchtemessgerät, z.B. Mettler Toledo HR73, bei 85°C über 10 min gemessen.

### Beispiel 1: Titrationsversuche an Omeprazol-Pelletkernen mit verschiedenen Basenkomponenten

Zur Optimierung des Pelletkerns wurden wirkstofffreie Rezepturen bestehend aus Mannitol, Hydroxypropylcellulose und Natriumlaurylsulfat mit verschiedenen Basenkomponenten versetzt und anschließend mit 0,1 N HCl titriert. Hierbei kamen unterschiedliche Mengen an Na₂HPO₄, MgCO₃ und Meglumine zum Einsatz.

Ziel der Untersuchungen war es, eine Base mit guter Pufferwirkung für den Pelletkern auszuwählen, um eine Degradation des Omeprazols beim Eindringen geringer Säuremengen während der Magenpassage (bzw. während der Magensaftresistenzprüfung) zu vermeiden. Weiterhin sollte nach Öffnen des magensaftresistenten Polymers ein leicht alkalischer pH-Wert vorliegen um eine mögliche Degradation des Wirkstoffs in vivo zu vermeiden.

Für Na₂HPO₄ wurde ein Bereich von 0,5 - 1,0 g untersucht. Auf höhere Mengen wurde verzichtet, da Na₂HPO₄ hygroskopische Eigenschaften hat, die sich nachteilig auf die Omeprazolstabilität auswirken können. Für MgCO₃ (erhältlich z.B. von Merck KGaA, Darmstadt, Deutschland bzw. von Magnesia, Lüneburg, Deutschland) wurde ein Bereich von 1 g bis 10 g und für Meglumine (N-Methyl-D-Glucamin) von 1 g bis 5 g (jeweils bezogen auf 79 g Pelletkerne ohne Wirkstoff) untersucht.

Zu Vergleichszwecken wurde auch eine Rezeptur ohne Base im Pelletkern in die Versuchsserie einbezogen.

Die Ergebnisse, die in Tabellen 1a und 1b dargestellt sind, lassen sich wie folgt zusammenfassen:
- Die Rezeptur ohne Base zeigt keinerlei puffernde Wirkung; der pH-Wert nach Suspendieren in Wasser liegt nach ca. 15 min Rühren im leicht sauren Bereich (ca. pH 6,1).
- Die Zugabe von MgCO₃ zur Hilfsstoffmischung bewirkt ein Ansteigen des pH-Wertes nach Suspendieren in Wasser auf ca. pH 9,5 bis 10. Während sich die pH-Werte der Rezepturen mit 1 g bis 10 g an MgCO₃ in Wasser nur geringfügig unterscheiden, ist ein deutlicher Anstieg der Pufferwirkung bei höherer Basenmenge festzustellen. Es können deutlich höhere Säuremengen neutralisiert werden, bevor ein Abfallen des pH-Wertes eintritt.
- Die Zugabe von Na₂HPO₄ zur Hilfsstoffmischung bewirkt ebenfalls eine Alkalisierung der Suspension. Die Werte sind mit ca. pH 8,0 - 8,5 etwas tiefer als bei MgCO₃, und auch die puffernde Wirkung ist deutlich schwächer ausgeprägt.
- Meglumine zeigt ein ähnliches Verhalten wie MgCO₃ mit pH-Werten der wässrigen Suspension von ca. pH 10 und vergleichbarer puffernder Wirkung.

**Tabelle 1a. Pufferwirkung verschiedener Basen**

| | **Ohne Base** | | **MgCO₃ Rezepturen** | | | | | | | |
|---|---|---|---|---|---|---|---|---|---|---|
| **Hilfsstoff** | **Menge [g]** | **Menge [Gew.-%]** | **Menge [g]** | **Menge [Gew.-%]** | **Menge [g]** | **Menge [Gew.-%]** | **Menge [g]** | **Menge [Gew.-%]** | **Menge [g]** | **Menge [Gew.-%]** |
| Mannitol | 78,00 | 98,7 | 77,00 | 97,5 | 73,00 | 92,4 | 68,00 | 86,1 | 68,00 | 86,1 |
| MgCO₃ schwer | - | - | 1,00 | 1,27 | 5,00 | 6,3 | 10,00 | 12,7 | - | - |
| MgCO₃ leicht | - | - | - | - | - | - | - | - | 10,00 | 12,7 |
| Hydroxypropylcellulose | 0,50 | 0,63 | 0,50 | 0,63 | 0,50 | 0,63 | 0,50 | 0,63 | 0,50 | 0,63 |
| Natriumlaurylsulfat | 0,50 | 0,63 | 0,50 | 0,63 | 0,50 | 0,63 | 0,50 | 0,63 | 0,50 | 0,63 |
| Summe Hilfsstoffe | 79,00 | 100 | 79,00 | 100 | 79,00 | 100 | 79,00 | 100 | 79,00 | 100 |
| pH (10 %-ige Suspension) | 6,12 | | 9,69 | | 9,58 | | 9,87 | | 9,87 | |
| Verbrauch 0,1N HCl bis etwa pH 8,0 | 0 | | ca. 2,5 ml | | 10 ml | | > 10 ml | | > 10 ml | |
| Verbrauch 0,1N HCl bis etwa pH 7,0 | 0 | | < 5 ml | | n.d. | | n.d. | | n.d | |

| | | | | | | | | | | |
|---|---|---|---|---|---|---|---|---|---|---|
| n.d.: nicht bestimmt | | | | | | | | | | |

**Tabelle 1b. Pufferwirkung verschiedener Basen**

| | **Na₂HPO₄ Rezepturen** | | | | | | **Meglumine Rezepturen** | | | |
|---|---|---|---|---|---|---|---|---|---|---|
| **Hilfsstoff** | **Menge [g]** | **Menge [Gew.-%]** | **Menge [g]** | **Menge [Gew.-%]** | **Menge [g]** | **Menge [Gew.-%]** | **Menge [g]** | **Menge [Gew.-%]** | **Menge [g]** | **Menge [Gew.-%]** |
| Mannitol | 77,50 | 98,1 | 77,00 | 97,5 | 77,00 | 97,5 | 77,00 | 97,5 | 73,00 | 92,4 |
| Na₂HPO₄ x 2H₂O | 0,50 | 0,63 | 1,00 | 1,27 | - | - | - | - | - | - |
| Na₂HPO₄ (wasserfrei) | - | - | - | - | 1,00 | 1,27 | - | - | - | - |
| Meglumine | - | - | - | - | - | - | 1,00 | 1,27 | 5,00 | 6,3 |
| Haydroxypropylcellulose | 0,50 | 0,63 | 0,50 | 0,63 | 0,50 | 0,63 | 0,50 | 0,63 | 0,50 | 0,63 |
| Natriumlaurylsulfat | 0,50 | 0,63 | 0,50 | 0,63 | 0,50 | 0,63 | 0,50 | 0,63 | 0,50 | 0,63 |
| Summe Hilfsstoffe | 79,00 | 100 | 79,00 | 100 | 79,00 | 100 | 79,00 | 100 | 79,00 | 100 |
| pH (10 %-ige Suspension) | 8,19 | | 8,34 | | 8,34 | | 9,85 | | 10,06 | |
| Verbrauch 0,1N HCl bis etwa pH 8,0 | < 0,03 ml | | 0,04 ml | | 0,03 ml | | 2,4 ml | | 12 ml | |
| Verbrauch 0,1N HCl bis etwa pH 7,0 | 0,25 ml | | 0,46 ml | | 0,54 ml | | 2,6 ml | | 12,4 ml | |

### Beispiel 2. Omeprazol-Pelletausbeuten in Abhängigkeit der Prozessbedingungen / Produktionsmaßstab

Der Einfluss verschiedener Prozessparameter bei der Herstellung der Pelletkerne (Zugabemenge an Isopropanol (Granuliermittel), Granulierzeit, Mischergeschwindigkeit) auf die Pelletausbeute wurde im Produktionsmaßstab untersucht (gesamte Feststoffmenge in der Charge 300 kg).

Die Ergebnisse sind in der folgenden Tabelle zusammengefasst:

| **charge** | **Granuliermittelmenge (Isopropanol) (kg)** | **Granulierzeit (min)** | **Mischer (U/min)** | **Ausbeute Pellets 700 - 1250 µm (kg)** |
|---|---|---|---|---|
| A | 50 - 53 | 25 - 30 | 40 | 10 - 20 |
| B | 50 - 53 | 17 - 19 | 40 | 50 - 60 |
| C | 56 - 59 | 10 - 15 | 50 | 65 - 75 |
| D | 56 - 59 | 10 - 15 | 60 | 80 - 90 |
| E | 62 - 65 | 9 - 10 | 65 | 120 - 140 |
| F | 62 - 65 | 5 - 8 | 70 - 80 | 170 - 200 |

Es zeigt sich, dass die Pelletausbeute im besonders wichtigen Bereich von 700 µm - 1250 µm insbesondere durch Verkürzung der Granulierzeit auf kleiner als 20 Minuten (insbesondere auf kleiner oder gleich 8 Minuten), durch Erhöhung der Granuliermittelmenge sowie durch Erhöhung der Mischergeschwindigkeit bei der Granulierung auf größer oder gleich 50 U/min, insbesondere auf größer oder gleich 65 U/min, deutlich gesteigert werden kann.

### Beispiel 3. Herstellungsbeispiele für Omeprazol-Pellets

Rezepturen mit unterschiedlichen Anteilen an Wirkstoff und Hilfsstoffen können nach dem beschriebenen Verfahren gefertigt werden. Die folgende **Tabelle 2** gibt Rezepturbeispiele in Bezug auf die Zusammensetzung des Pelletkerns, der Schutzschicht und der magensaftresistenten Schicht. Die angegebenen Rezepturbeispiele beziehen sich auf Ansatzgrößen von 1 kg Pelletkernen.

**Tabelle 2. Rezepturbeispiele Omeprazol-Pellets**

| **Rezepturbeispiel** | **1** | **2** | **3** | **4** | **5** |
|---|---|---|---|---|---|
| **Pelletkern** | | | | | |
| Omeprazol | 100 | 180 | 180 | 360 | 540 |
| Mannit | 874 | 760 | 755 | 515 | 330 |
| Magnesiumcarbonat | 20 | 50 | 50 | 100 | 100 |
| Hydroxypropylcellulose | 1 | 5 | 5 | 5 | 10 |
| Natriumlaurylsulfat | 5 | 5 | 10 | 20 | 20 |

| **Zwischenschicht** | | | | | |
|---|---|---|---|---|---|
| Hydroxypropylcellulose (6cP) | 3,12 | 3,12 | 4,35 | 4,35 | 4,35 |
| Magnesiumcarbonat | 4,87 | 4,87 | --- | --- | --- |
| Talkum | 3,12 | 3,12 | 4,35 | 4,35 | 4,35 |

| **Magensaftresistente Schicht** | | | | | |
|---|---|---|---|---|---|
| Eudragit L30-D55 (Feststoff) | 35,01 | 35,01 | 31,06 | 24,15 | 31,06 |
| NaOH zur pH-Einstellung auf pH 5,2 | q.s. | q.s. | --- | q.s. | --- |
| Talkum | 7,00 | 7,00 | 12,42 | 9,66 | 12,42 |
| PEG 6000 | 3,50 | 3,50 | --- | 2,42 | --- |
| Triethylcitrat | --- | --- | 3,11 | --- | 3,11 |
| Titandioxid | 2,10 | 2,10 | --- | 2,10 | --- |

Die abgewogenen Komponenten des Pelletkerns werden in einen pharmazeutisch gebräuchlichen Schnellmischer überführt und gemischt. Isopropanol wird unter kontinuierlichem Mischen/Granulieren zu der Pulvermischung im Mischer gegeben bis sich Pellets mit der erforderlichen Qualität (Rundheit, Durchmesser ca. 1 mm) bilden. Die Mischzeit beträgt 1 bis 10 min, üblicherweise 5 bis 8 min, um eine geeignete Qualität und Ausbeute zu erreichen.

Die Isopropanol feuchten Pellets werden anschließend in einem Trockner bei einer Zulufttemperatur von ca. 50 - 70°C für ca. 30 - 60 min getrocknet bis ein Trocknungsverlust von kleiner 1 Gew.-%, bevorzugt kleiner 0,3 Gew.-% erhalten wird. Die Pellets mit dem gewünschten Durchmesser kleiner 2 mm, insbesondere diejenigen mit einem Durchmesser von 700 bis 1250 µm werden anschließend abgesiebt.

Die Pellets werden anschließend in der Wirbelschicht mit der wässrigen Suspension der Zwischenschicht (Herstellung nach üblichen pharmazeutischen Verfahren), und nach einer Zwischentrockenphase mit der wässrigen Suspension der magensaftresistenten Schicht (Herstellung nach üblichen pharmazeutischen Verfahren) unter geeigneten Prozessbedingungen lackiert (befilmt) und anschließend getrocknet, bis die Restfeuchte (Wassergehalt) kleiner oder gleich 1,5 Gew.-%, vorzugsweise kleiner oder gleich 1 Gew.-% ist.

Die magensaftresistent befilmten Pellets werden anschließend gesiebt und in Hartgelatinekapseln abgefüllt.

### Beispiel 4. Freisetzung und Stabilität der Omeprazol-Pellets

### Pelletkerne

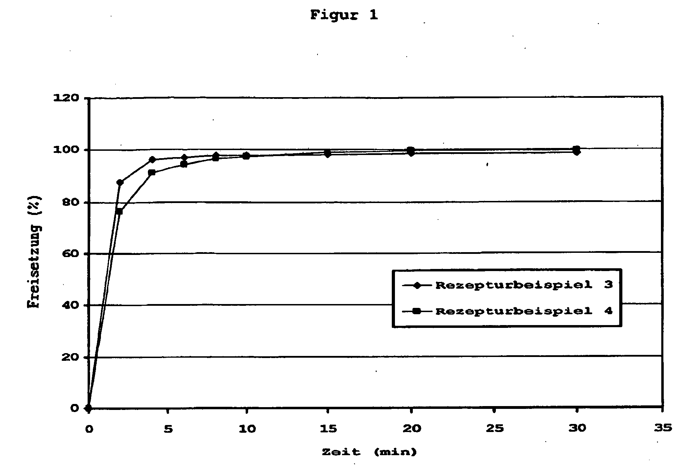

Die **Auflösungsgeschwindigkeit** des Omeprazols aus den reinen Pelletkernen wurde unter Verwendung einer Blattrührerapparatur nach dem Europäischen Arzneibuch Version 6, bei 100 U/min und 37°C, in Phosphatpuffer bei einer Lösungsmittelmenge von 900 mL und einem pH von 6,8 gemessen. Die Bestimmung des gelösten (oder freigesetzten) Omeprazols erfolgte unter Verwendung eines Freisetzungsmessgeräts der Fa. Distec (Premiere 5100 Dissolution System mit Agilent 8453 UV-online System) mit UV-online Absorptionsmessung bei 301 nm und Hintergrundkorrektur bei 345 nm unter Verwendung von Quarzküvetten mit einer Schichtdicke von 5 mm bis 10 mm. Die Auflösungsgeschwindigkeit von Omeprazol für die Rezepturbeispiele 3 und 4 ist in **Figur 1** graphisch dargestellt.

Wie aus dieser Figur ersichtlich zerfallen die Pelletkerne schnell und setzen den Wirkstoff bei der Freisetzungsprüfung sehr schnell frei, d.h. innerhalb von 10 min werden über 95 % des Omeprazols gelöst.

Um die **Stabilität** der Pelletkerne zu bestimmen wurden gemäß Rezepturbeispiel 3 hergestellte Pelletkerne unter Stressbedingungen eingelagert und anschließend die Auflösungsgeschwindigkeit von Omeprazol wie oben dargelegt untersucht.

Die folgenden Stressbedingungen wurden angewandt:
- Rzp 3: Rezepturbeispiel 3, unbehandelt
- WO2/40: 40°C, 75 % Luftfeuchtigkeit, offenes Glasgefäß, 2 Wochen
- WO2/60: 60°C, geschlossenes Glasgefäß, 2 Wochen
- M01/25: 25°C, 60 % Luftfeuchtigkeit, offenes Glasgefäß, 1 Monat
- M01/40: 40°C, 75 % Luftfeuchtigkeit, offenes Glasgefäß, 1 Monat
- WO2/40 PE: 40°C, 75 % Luftfeuchtigkeit, geschlossene PE-Flasche & Trockenmittel (Silicagel), 2 Wochen
- M03/25: 25°C, 60 % Luftfeuchtigkeit, offenes Glasgefäß, 3 Monate

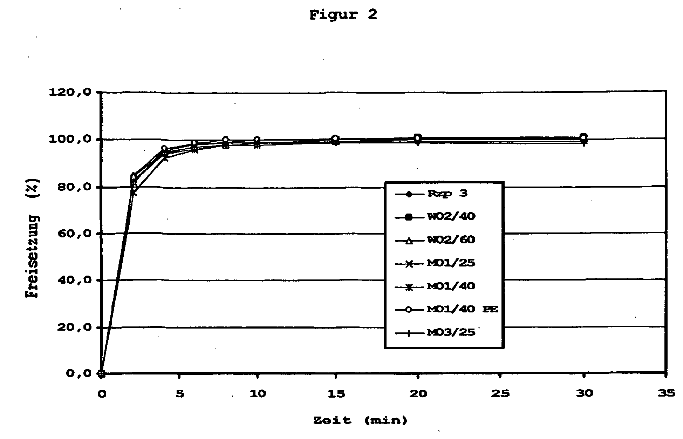

Die Auflösungsgeschwindigkeiten ergeben sich aus **Tabelle 3** und sind in **Figur 2** graphisch veranschaulicht.

**Tabelle 3. Auflösungsgeschwindigkeit Rezepturbeispiel 3**

| | Freisetzung (%) | | | | | | |
|---|---|---|---|---|---|---|---|
| Zeit (min) | Rzp 3 | WO2/40 | WO2/60 | M01/25 | M01/40 | M01/40 PE | M03/25 |
| 0 | 0,0 | 0,0 | 0,0 | 0,0 | 0,0 | 0,0 | 0,0 |
| 2 | 84,6 | 82,5 | 84,4 | 81,7 | 77,1 | 81,5 | 82,0 |
| 4 | 96,2 | 94,0 | 94,9 | 95,5 | 92,4 | 96,4 | 94,5 |
| 6 | 98,5 | 96,3 | 97,0 | 98,5 | 96,0 | 98,9 | 97,1 |
| 8 | 99,0 | 97,9 | 98,0 | 99,5 | 97,9 | 100,0 | 98,2 |
| 10 | 99,1 | 99,0 | 99,0 | 100,0 | 98,0 | 100,3 | 98,7 |
| 15 | 99,3 | 100,3 | 100,1 | 100,2 | 99,3 | 100,5 | 99,0 |
| 20 | 99,2 | 100,8 | 100,7 | 100,4 | 100,0 | 100,5 | 98,9 |
| 30 | 99,3 | 101,1 | 100,7 | 100,4 | 100,1 | 100,4 | 98,3 |

Wie aus diesen Untersuchungen ersichtlich besitzen die gemäß Rezepturbeispiel 3 hergestellten Pelletkerne eine ausgezeichnete Stabilität und setzen den Wirkstoff auch nach Einlagerung unter Stressbedingungen sehr schnell innerhalb von 10 min vollständig frei. Diese ausgezeichnete Stabilität könnte durch den chemischen Aufbau und die geringe Restfeuchte der Pelletkerne bedingt sein.

### Vergleich

Um den Einfluss der in den Pelletkernen verwendeten Inhaltsstoffe auf die Stabilität der Pellets zu ermitteln wurden Vergleichsrezepturen gemäß dem Stand der Technik hergestellt. Die Zusammensetzung der Pelletkerne für das Vergleichsrezepturbeispiel V1 wurde gemäß europäischer Patentanmeldung EP-A-0 247 983 ausgewählt. Sie unterscheidet sich von den zuvor genannten Rezepturbeispielen 1 bis 5 insbesondere in der verwendeten Basenkomponente Dinatriumhydrogenphosphat anstelle von Magnesiumcarbonat. Die Pelletkerne gemäß Vergleichsrezepturbeispiel V1 wurden unter analogen Prozessbedingungen wie die Rezepturbeispiele 1 bis 5 mit einer Schutzschicht und einer magensaftresistenten Schicht befilmt. Die Zusammensetzung ist in der folgenden **Tabelle 4** dargestellt. Das angegebene Rezepturbeispiel bezieht sich auf eine Ansatzgröße von 1 kg Pelletkerne.

**Tabelle 4. Vergleichsrezepturbeispiel Omeprazol-Pellets**

| **Rezepturbeispiel** | **V1** |
|---|---|
| **Pelletkern** | |
| Omeprazol | 100 |
| Mannit | 790 |
| Laktose wasserfrei | 40 |
| Cellulose mikrokristallin | 20 |
| Dinatriumhydrogenphosphat Dihydrat | 10 |
| Hydroxypropylcellulose | 30 |
| Natriumlaurylsulfat | 10 |

| **Zwischenschicht** | |
|---|---|
| Hydroxypropylcellulose (6cp) | 4,35 |
| Talkum | 4,35 |

| **Magensaftresistente Schicht** | |
|---|---|
| Eudragit L30-D55 (Feststoff) | 24,15 |
| NaOH zur pH-Einstellung auf pH 5,2 | q.s. |
| Talkum | 9,66 |
| PEG 6000 | --- |
| Triethylcitrat | 2,42 |
| Titandioxid | 2,10 |

Die Rezepturbeispiele 2 und 3 und die Vergleichsrezeptur V1 wurden in einer Pelletmenge entsprechend 20 mg Omeprazol in Hartgelatinekapseln abgefüllt und zu einer Stressstabilitätsuntersuchung über 2 Wochen eingelagert. Die Einlagerung erfolgte offen bei 40°C / 75% relativer Feuchte sowie verschlossen in einer Glasflasche mit Schraubverschluss bei 60°C. Die Pellets wurden anschließend hinsichtlich der Gesamtmenge der Verunreinigungen HPLC-analytisch im Vergleich zum Startwert untersucht. Die erhaltenen Ergebnisse sind in der folgenden **Tabelle 5** dargestellt.

**Tabelle 5. Stabilitätsuntersuchung von Omeprazol-Pellets im Vergleich**

| **Einlagerungsbedingung** | **Gesamtmenge Verunreinigungen (Flächen %)** | | |
|---|---|---|---|
| | Rezepturbeispiel 2 | Rezepturbeispiel 3 | Vergleichsbeispiel V1 |
| Start | 0,10 | 0,10 | 0,06 |
| 2 Wochen offen 40°C/75% r.F. | 0,45 | 0,37 | 6,01 |
| 2 Wochen verschlossen Glasflasche, 60°C | 0,47 | 0,51 | 1,85 |

Wie aus dieser Tabelle ersichtlich sind die Rezepturbeispiele 2 und 3, die Magnesiumcarbonat enthalten, deutlich stabiler als Beispiel V1, in dem Natriumdihydrogenphosphat verwendet wurde.

### Magensaftresistent lackierte (befilmte) Pellets

Da Omeprazol in saurem Medium nicht stabil ist, wurden verschiedene Mengen an magensaftresistenter Schicht (Eudragit L30-D55) auf die Pelletkerne nach Rezepturbeispiel 3 aufgetragen und die Stabilität der beschichteten Kerne wie folgt untersucht.

Zur Ermittlung der Magensaftresistenz wurden mit unterschiedlichen Filmdicken befilmte (lackierte) Omeprazol-haltige Kerne unter Verwendung einer Blattrührerapparatur nach dem Europäischen Arzneibuch Version 6, bei 100 U/min und 37°C, zwei Stunden einer Lösungsmittelmenge von 500 mL mit pH 1,2 (salzsaures Medium) bzw. pH 4,5 (Natriumacetatpuffer) ausgesetzt. Anschließend wurde das Freisetzungsmedium dekantiert, die Kerne isoliert und unter Anwendung einer hochdruckflüssigkeitschromatographischen Gehaltsbestimmung mit UV-Absorptionsmessung bei 280 nm die Menge intakten Omeprazols bestimmt. Das prozentuale Verhältnis zwischen dem Omeprazolgehalt nach zwei Stunden Freisetzungsprüfung und dem Omeprazolgehalt vor der Freisetzungsprüfung wird als Magensaftresistenz (%) bezeichnet. Die Ergebnisse sind in **Tabelle 6** dargestellt.

**Tabelle 6. Stabilität von magensaftresistent beschichteten Omeprazol-Kernen**

| **MSR-Lackauftrag (%)*** | **Magensaftresistenz (%)** | |
|---|---|---|
| | **pH 1,2** | **pH 4,5** |
| 10,0 | < 50 | < 50 |
| 15,0 | 72 | < 50 |
| 20,0 | 97 | 91 |
| 25,0 | 96 | 93 |
| 27,5 | 97 | 97 |
| 30,0 | 100 | 100 |

| | | |
|---|---|---|
| *MSR-Lackauftrag (%): Massenanteil magensaftresistenter Schicht (Lack) an Gesamtmasse (Kern, Zwischenschicht und magensaftresistente Schicht) | | |

Es zeigt sich, dass zum Erzielen der gewünschten Magensaftresistenz ein Auftrag von 20 % oder mehr, bevorzugt 25 % oder mehr magensaftresistenter Lackschicht erforderlich ist, um auch eine entsprechende Magensaftresistenz zu erzielen. Schichtdicken von mindestens 20 % setzen so gut wie keinen Wirkstoff frei und schützen den Wirkstoff vor dem chemischen Angriff des sauren Mediums.

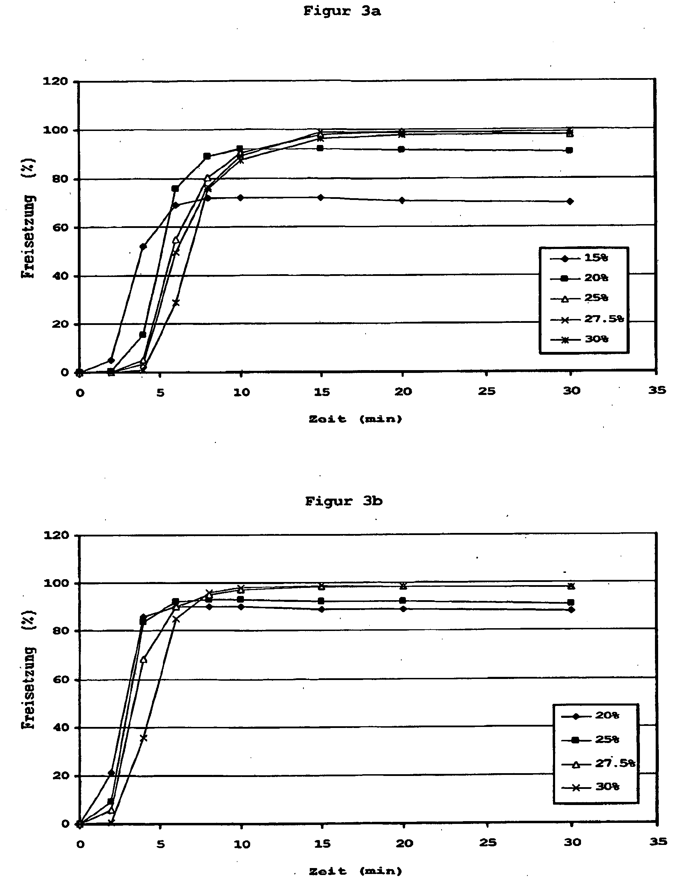

Anschließenden wurde der pH des Auflösungsmediums durch Zugabe von 400 ml entsprechend konzentrierter Phosphatpufferlösung, auf pH 6,8 (der das neutrale pH Milieu des Darms widerspiegelt) verändert. Wie aus **Figur 3a** und **Figur 3b** ersichtlich, wird der Wirkstoff bei pH 6,8 bei einem Lackauftrag von mindestens 25 % innerhalb von 10 min zu über 80 % und innerhalb von 20 min zu über 90 % freigesetzt. Der pH-Wert während der Magensaftresistenzprüfung hat lediglich einen geringen Einfluss auf die Auflösungsgeschwindigkeit.

### Referenz-Beispiel 5. Herstellungsbeispiele für Diclofenac-Pellets

Die folgende **Tabelle 7** gibt Rezepturbeispiele in Bezug auf die Zusammensetzung von Diclofenac-haltigen Pelletkernen mit einer magensaftresistenten bzw. retardierenden Schicht. Die angegebenen Rezepturbeispiele beziehen sich auf Ansatzgrößen von 1 kg Pelletkernen.

**Tabelle 7. Rezepturbeispiele für Diclofenac-Pellets**

| **Rezepturbeispiel** | **6** | **7** |
|---|---|---|
| **Pelletkern** | | |
| Diclofenac-Natrium | 500 | 500 |
| Microcrystalline Cellulose | 440 | 440 |
| Polyvinylpyrrolidone | 40 | 40 |
| Siliziumdioxid hochdispers | 20 | 20 |
| | | |

| **Magensaftresistente Schicht** | | |
|---|---|---|
| Eudragit L30-D55 (Feststoff) | 200 | --- |
| NaOH zur pH-Einstellung auf pH 5,2 | q.s. | --- |
| Propylenglykol | 20 | --- |
| Talkum | 100 | --- |
| | | |

| **Retardierende Schicht** | | |
|---|---|---|
| Eudragit RS100 | --- | 40 |
| Eudragit RL100 | --- | 8,0 |
| Triethylcitrat | --- | 4,8 |
| Talkum | --- | 36 |

Die abgewogenen Komponenten des Pelletkerns werden in einen pharmazeutisch gebräuchlichen Schnellmischer überführt und gemischt. Isopropanol wird unter kontinuierlichem Mischen/Granulieren zu der Pulvermischung im Mischer gegeben bis sich Pellets mit der erforderlichen Qualität (Rundheit, Durchmesser ca. 1 mm) bilden. Die Mischzeit beträgt 1 bis 10 min, üblicherweise 5 bis 8 min, um eine geeignete Qualität und Ausbeute zu erreichen.

Die Isopropanol feuchten Pellets werden anschließend in einem Trockner bei einer Zulufttemperatur von ca. 50 - 70°C für ca. 30 - 60 min getrocknet bis ein Trocknungsverlust kleiner 2 Gew.-%, noch besser kleiner 1,0 Gew.-% erhalten wird. Die Pellets mit dem gewünschten Durchmesser kleiner 2 mm, insbesondere diejenigen mit einem Durchmesser von 700 bis 1250 µm werden anschließend abgesiebt.

Für die **magensaftresistente Lackierung** werden die Pellets anschließend in der Wirbelschicht mit der wässrigen Suspension des magensaftresistenten Polymers, in der Propylenglykol gelöst und Talkum suspendiert wird (Herstellung nach üblichen pharmazeutischen Verfahren), unter den oben beschriebenen Prozessbedingungen lackiert (befilmt) und anschließend getrocknet, bis die Restfeuchte (Wassergehalt) kleiner oder gleich 4,0 Gew.-%, vorzugsweise kleiner oder gleich 3,0 Gew.-%, besonders bevorzugt kleiner oder gleich 2,5 Gew.-% ist.

Für die **retardierende Lackierung** werden die Pellets in der Wirbelschicht mit einer organischen Lösung (Isopropanol/Aceton) der Retardpolymere, in der Triethylcitrat gelöst und Talkum suspendiert wird (Herstellung nach üblichen pharmazeutischen Verfahren), unter oben beschriebenen Prozessbedingungen lackiert (befilmt) und anschließend getrocknet, bis die Restfeuchte (Wassergehalt) kleiner oder gleich 2 Gew.-%, vorzugsweise kleiner oder gleich 1,5 Gew.-%, besonders bevorzugt kleiner oder gleich 1,0 Gew.-% ist.

Die befilmten Pellets werden anschließend gesiebt, gemischt und in Hartgelatinekapseln abgefüllt.

### Referenz-Beispiel 6. Freisetzung der Diclofenac-Pellets

### Freisetzungskurven Diclofenac-Pellets

Die Auflösungsgeschwindigkeiten der Diclofenac-Pellets der Rezepturbeispiele 6 und 7 und einer Mischung davon wurde bei verschiedenen pH-Werten untersucht. Hierzu wurden magensaftresistent befilmte Pellets enthaltend 25 mg Diclofenac-Natrium, retardierend lackierte Pellets enthaltend 50 mg Diclofenac-Natrium, sowie eine Mischung aus magensaftresistent lackierten Pellets enthaltend 25 mg Diclofenac-Natrium und retardierend lackierten Pellets enthaltend 50 mg Diclofenac-Natrium untersucht.

Es wurde eine Drehkörbchenapparatur nach dem Europäischen Arzneibuch Version 6 mit einer Lösungsmittelmenge von 1000 ml bei 100 U/min und 37 °C verwendet. Nach 2 Stunden Freisetzungsprüfung unter Verwendung von salzsaurem Medium (pH 1,2) erfolgte ein Wechsel des Freisetzungsmediums zu Phosphatpuffer (pH 6,8) und die Auflösungsgeschwindigkeit wurde bis zu weitere 6 Stunden untersucht. Die Bestimmung des gelösten Diclofenac erfolgte mit UV-Absorptionsmessung bei 281.

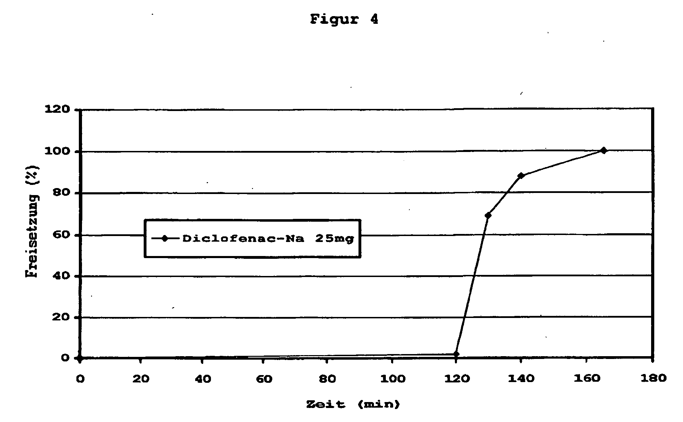

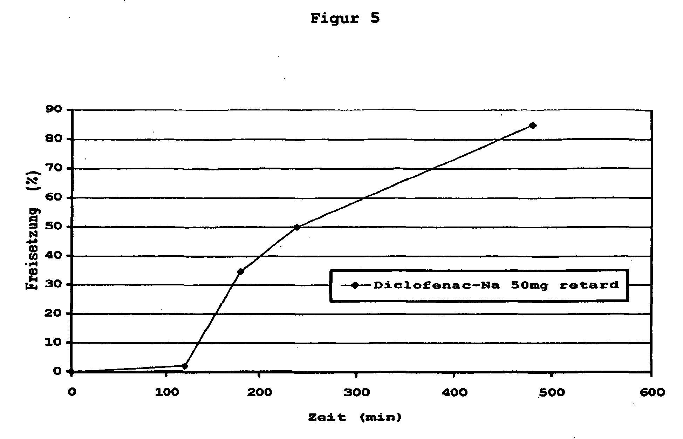

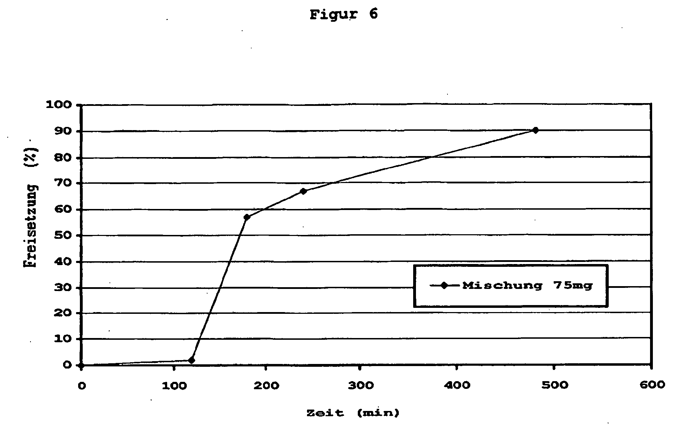

Die Auflösungsgeschwindigkeiten ergeben sich aus **Tabelle 8, Tabelle 9** und **Tabelle 10** und sind in **Figur 4****,** **Figur 5** bzw. **Figur 6** graphisch veranschaulicht.

**Tabelle 8. Auflösungsgeschwindigkeiten von Rezepturbeispiel 6**

| | pH 1,2 | pH 1,2 | pH 6,8 | pH 6, 8 | pH 6,8 |
|---|---|---|---|---|---|
| Zeit (min) | 0 | 120 | 130 | 140 | 165 |
| Freisetzung (%) | 0 | 2 | 69 | 88 | 100 |

**Tabelle 9. Auflösungsgeschwindigkeiten von Rezepturbeispiel 7**

| | **pH 1,2** | **pH 1,2** | **pH 6,8** | **pH 6,8** | **pH 6,8** |
|---|---|---|---|---|---|
| Zeit (min) | 0 | 120 | 180 | 240 | 480 |
| Freisetzung (%) | 0 | 2 | 35 | 50 | 85 |

**Tabelle 10. Auflösungsgeschwindigkeiten einer Mischung aus Rezepturbeispielen 6 und 7**

| | **pH 1,2** | **pH 1,2** | **pH 6,8** | **pH 6,8** | **pH 6,8** |
|---|---|---|---|---|---|
| Zeit (min) | 0 | 120 | 180 | 240 | 480 |
| Freisetzung (%) | 0 | 2 | 57 | 67 | 90 |

### Pharmakologische Bedeutung des Freisetzungsprofils

Aus den Freisetzungskurven kann man folgende Informationen ableiten:
- Bei pH 1,2 erfolgt nahezu keine Freisetzung des Diclofenac-Natrium
- Nach pH-Wechsel auf pH 6,8 erfolgt aus den magensaftresistent umhüllten Pellets die vollständige Freisetzung des Diclofenac innerhalb von 45 min
- Nach pH-Wechsel auf pH 6,8 erfolgt aus den verzögert umhüllten Pellets die Freisetzung kontinuierlich über einen Zeitraum von mehr als 6 Stunden
- Nach pH-Wechsel auf pH 6,8 zeigt die Pelletmischung eine Addition oben genannter Diclofenac-Natrium Freisetzungen mit dem Vorteil der schnellen Freisetzung des magensaftresistenten Anteils (initiale Dosis) und der langsamen Freisetzung des retardierten Anteils (kontinuierliche Dosis) über einen Zeitraum von 6 Stunden oder mehr.

Hierdurch wird in vivo ein schneller Wirkungseintritt (über die Initialdosis des magensaftresistenten Anteils) und ein Aufrechterhalten geeigneter Blutspiegel über einen längeren Zeitraum (über den retardierten Anteil) gewährleistet, was pharmakologisch besonders vorteilhaft insbesondere zur Behandlung rheumatoider Beschwerden ist.

### Beispiel 7. Pharmazeutische Zubereitung enthaltend eine Kombination aus Omeprazol- und Diclofenac-Pellets

Eine Hartgelatinekapsel wurde mit erfindungsgemäßen magensaftresistent befilmten Omeprazol-Pellets (enthaltend insgesamt 20 mg Omeprazol), magensaftresistent befilmten Diclofenac-Natrium Pellets (enthaltend insgesamt 25 mg Diclofenac-Natrium) und mit retardierend freisetzenden Diclofenac-Natrium Pellets (enthaltend insgesamt 50 mg Diclofenac-Natrium) befüllt zum Erhalt einer pharmazeutischen Zusammensetzung, die eine Kombination beider Wirkstoffe enthält. Die magensaftresistent befilmten Omeprazol-Pellets wurden gemäß Beispiel 3 (Rezepturbeispiel 3) hergestellt. Die magensaftresistent befilmten und die retardiert freisetzenden Diclofenac-Natrium Pellets wurden gemäß Beispiel 5 (Rezepturbeispiele 6 bzw. 7) hergestellt.

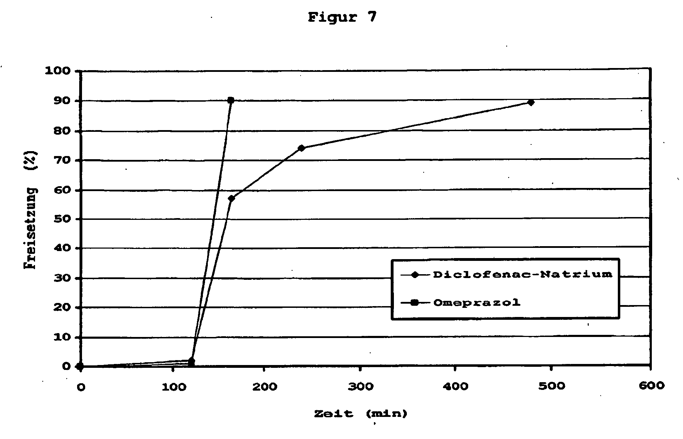

Anschließend wurde die Freisetzung der Wirkstoffe bei verschiedenen pH-Werten untersucht. Es wurde eine Drehkörbchenapparatur nach dem Europäischen Arzneibuch Version 6 mit einer Lösungsmittelmenge von 900 ml bei 100 U/min und 37 °C verwendet. Nach 2 Stunden Freisetzungsprüfung unter Verwendung von salzsaurem Medium (pH 1,2) erfolgte ein Wechsel des Freisetzungsmediums zu Phosphatpuffer (pH 6,8) und die Auflösungsgeschwindigkeit wurde über weitere 6 Stunden verfolgt. Die Bestimmung der beiden Wirkstoffe erfolgte nach hochdruckflüssigkeitschromatographischer Trennung mittels UV-Absorptionsmessung bei 280 nm. Die Bestimmung des nach 2 Stunden freigesetzten Omeprazols erfolgte indirekt über die Bestimmung des Omeprazolrestgehalts der isolierten Pellets in einer parallel durchgeführten Magensaftresistenzprüfung unter Verwendung identischer Versuchsbedingungen (pH 1,2 über 2 Stunden). Die Auflösungsgeschwindigkeiten sind in **Tabelle 11** angegeben und in **Figur 7** graphisch veranschaulicht.

**Tabelle 11. Auflösungsgeschwindigkeit Kombinationszubereitung**

| | **pH 1,2** | **pH 1,2** | **pH 6,8** | **pH 6,8** | **pH 6,8** |
|---|---|---|---|---|---|
| Zeit (min) | 0 | 120 | 165 | 240 | 480 |
| Freisetzung Omeprazol (%) | 0 | 1* | 90 | nicht bestimmt | nicht bestimmt |
| Freisetzung Diclofenac (%) | 0 | 2 | 57 | 74 | 89 |

| | | | | | |
|---|---|---|---|---|---|
| * Angabe bezogen auf den Omeprazolrestgehalts bei der Magensaftresistenzprüfung bei gleichen Versuchsbedingungen (pH 1,2 über 2 Stunden) | | | | | |

### Beispiel 8. Magensaftresistent befilmte Pellets enthaltend eine Kombination aus Omeprazol und Diclofenac

Gemäß der in Beispiel 3 genannten Herstellungsweise können auch magensaftresistent befilmte Pellets, die eine Kombination aus Omeprazol und Diclofenac-Natrium mit unterschiedlichen Mengenanteilen enthalten, hergestellt werden. Die folgende **Tabelle 12** gibt Rezepturbeispiele in Bezug auf die Zusammensetzung des Pelletkerns, der Schutzschicht und der magensaftresistenten Schicht. Die angegebenen Rezepturbeispiele beziehen sich auf Ansatzgrößen von 1 kg Pelletkernen.

**Tabelle 12. Rezepturbeispiele von magensaftresistent befilmten Pellets, die Omeprazol und Diclofenac enthalten**

| **Rezepturbeispiel** | **8** | **9** | **10** |
|---|---|---|---|
| **Pelletkern** | | | |
| Omeprazol | 250 | 180 | 90 |
| Diclofenac-Natrium | 250 | 225 | 270 |
| Mannit | 430 | 520 | 560 |
| Magnesiumcarbonat | 50 | 50 | 50 |
| Hydroxypropylcellulose | 10 | 15 | 25 |
| Natriumlaurylsulfat | 10 | 10 | 5 |

| **Zwischenschicht** | | | |
|---|---|---|---|
| Hydroxypropylcellulose (6cP) | 4,35 | 4,35 | 3,12 |
| Magnesiumcarbonat | --- | --- | 4,87 |
| Talkum | 4,35 | 4,35 | 3,12 |

| **Magensaftresistente Schicht** | | | |
|---|---|---|---|
| Eudragit L30-D55 (Feststoff) | 31,06 | 31,06 | 35,01 |
| NaOH zur pH-Einstellung auf pH 5,2 | --- | --- | q.s. |
| Talkum | 12,42 | 12,42 | 7,00 |
| PEG 6000 | --- | --- | 3,50 |
| Triethylcitrat | 3,11 | 3,11 | --- |
| Titandioxid | --- | --- | 2,10 |

Die nach dem erfindungsgemäßen Herstellungsverfahren erhaltenen magensaftresistent befilmten Pellets werden gesiebt und in Hartgelatinekapseln abgefüllt. Anschließend werden magensaftresistent befilmte Pellets in einer Dosierung von 20 mg Omeprazol und 25 mg Diclofenac-Natrium, sowie in einer Dosierung von 10 mg Omeprazol und 25 mg Diclofenac-Natrium in die Hartgelatinekapseln gefüllt.

Die Freisetzung der Wirkstoffe aus den Darreichungsformen gemäß Rezepturbeispielen 9 und 10 wurde bei verschiedenen pH-Werten untersucht, analog zur Vorgehensweise wie sie in Beispiel 7 beschrieben ist (Drehkörbchenapparatur, 900 ml, 100 U/min, 37 °C). Nach 2 Stunden Freisetzungsprüfung erfolgte Mediumwechsel von pH 1,2 nach pH 6,8 und die Fortsetzung der Messung der Auflösungsgeschwindigkeit über weitere 45 Minuten. Die analytische Bestimmung der beiden Wirkstoffe erfolgte wie in Beispiel 7 beschrieben.

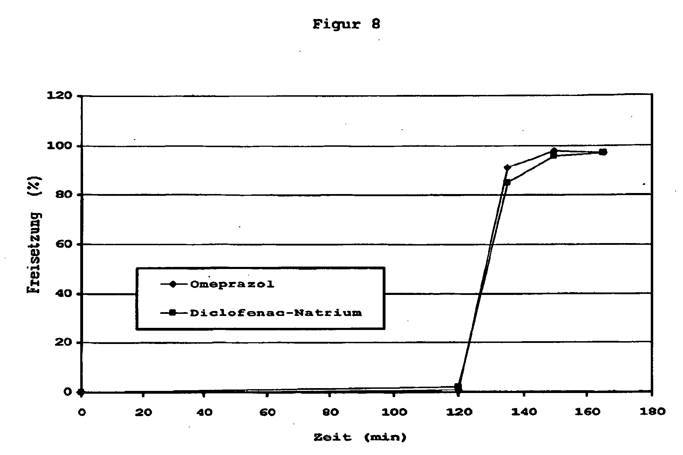

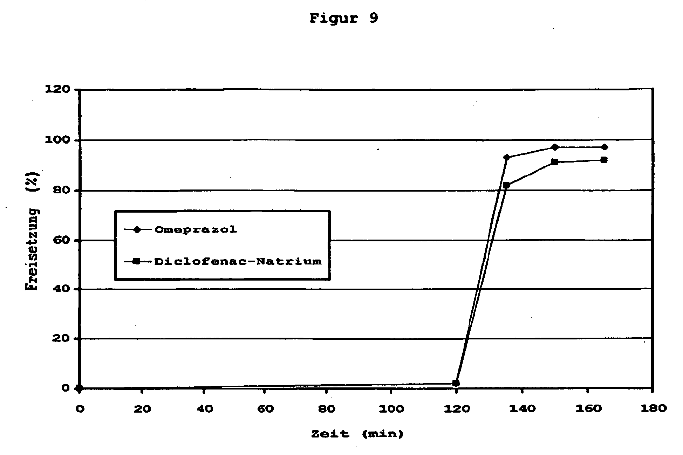

Die Auflösungsgeschwindigkeiten sind in **Tabelle 13** und **Tabelle 14** angegeben und in **Figur 8** bzw. **Figur 9** graphisch veranschaulicht.

**Tabelle 13. Auflösungsgeschwindigkeit einer Kombinationszubereitung umfassend 20 mg Omeprazol und 25 mg Diclofenac-Natrium**

| | **pH 1,2** | **pH 1,2** | **pH 6,8** | **pH 6,8** | **pH 6,8** |
|---|---|---|---|---|---|
| Zeit (min) | 0 | 120 | 135 | 150 | 165 |
| Freisetzung Omeprazol (%) | 0 | 1* | 91 | 98 | 97 |
| Freisetzung Diclofenac (%) | 0 | 2 | 85 | 96 | 97 |

| | | | | | |
|---|---|---|---|---|---|
| * Angabe bezogen auf den Omeprazolrestgehalt bei der Magensaftresistenzprüfung bei gleichen Versuchsbedingungen (pH 1,2 über 2 Stunden) | | | | | |

**Tabelle 14. Auflösungsgeschwindigkeit einer Kombinationszubereitung umfassend 10 mg Omeprazol und 25 mg Diclofenac-Natrium**

| | **pH 1,2** | **pH 1,2** | **pH 6,8** | **pH 6,8** | **pH 6,8** |
|---|---|---|---|---|---|
| Zeit (min) | 0 | 120 | 135 | 150 | 165 |
| Freisetzung Omeprazol (%) | 0 | 2* | 93 | 97 | 97 |
| Freisetzung Diclofenac (%) | 0 | 2 | 82 | 91 | 92 |

| | | | | | |
|---|---|---|---|---|---|
| * Angabe bezogen auf den Omeprazolrestgehalts bei der Magensaftresistenzprüfung bei gleichen Versuchsbedingungen (pH1,2 über 2 Stunden) | | | | | |

Die Freisetzung der beiden Wirkstoffe erfolgte nach dem pH-Wechsel sehr schnell und vollständig.

## Patentansprüche

1. Verfahren zur Herstellung einer pharmazeutischen Zubereitung, die einen PPI (Protonenpumpeninhibitor) in Form von sphärischen Granulaten (Pellets) enthält, umfassend die folgenden Schritte:
(a) Granulieren des PPI, mindestens einer basischen Komponente, mindestens eines pharmazeutisch annehmbaren Hilfsstoffes, und mindestens eines Alkohols, vorzugsweise von Ethanol und/oder Isopropanol, insbesondere von Isopropanol, unter Verwendung eines Schnellmischers, zum Erhalt von PPI-haltigen Kernen,
(b) Trocknen der Kerne,
(c) Überziehen der getrockneten Kerne mit einer inerten Zwischenschicht,
(d) Befilmen der überzogenen Kerne mit einer magensaftresistenten Schicht, und
(e) Trocknen der magensaftresistent befilmten Kerne auf eine Restfeuchte von kleiner oder gleich 1,5 Gew.-%, vorzugsweise kleiner oder gleich 1 Gew.-%, zum Erhalt von PPI-Pellets,
wobei die Rührflügelgeschwindigkeit des Schnellmischers größer oder gleich 50 U/min ist, vorzugsweise größer oder gleich 65 U/min ist, noch bevorzugter im Bereich von 70 bis 140 U/min liegt, die Granulierzeit vorzugsweise kleiner als 20 Minuten, noch bevorzugter kleiner oder gleich 10 Minuten, besonders bevorzugt kleiner oder gleich 8 Minuten und am bevorzugtesten 5 bis 8 Minuten ist, und das Granulieren (a) in Abwesenheit von Wasser geschieht.

2. Verfahren gemäß Anspruch 1, wobei der PPI ausgewählt ist aus Omeprazol, Esomeprazol, Lansoprazol, Pantoprazol und Rabeprazol, vorzugsweise aus Omeprazol.

3. Verfahren gemäß Anspruch 1 oder 2, wobei die pharmazeutische Zubereitung weiterhin ein NSAID (nichtsteroidales Antirheumatikum) in Form von Pellets enthält, wobei ein Teil des NSAID
(i) in Form eines Salzes, beispielsweise als Natrium-Salz, zusammen mit dem PPI gemäß Schritten (a) bis (e) granuliert, getrocknet, überzogen, befilmt und erneut getrocknet wird und/oder
(ii)in freier Form oder in Form eines Salzes, beispielsweise als Natrium-Salz, getrennt von dem PPI, zusammen mit mindestens einem pharmazeutisch annehmbaren Hilfsstoff und mindestens einem Alkohol, vorzugsweise von Ethanol und/oder Isopropanol, insbesondere von Isopropanol, unter Verwendung eines Schnellmischers, zum Erhalt von NSAID-haltigen Kernen granuliert, die Kerne getrocknet, mit einer magensaftresistenten Schicht befilmt und die magensaftresistent befilmten Kerne auf eine Restfeuchte von kleiner oder gleich 4,0 Gew.-%, vorzugsweise von kleiner oder gleich 3,0 Gew.-%, besonders bevorzugt von kleiner oder gleich 2,5 Gew.-% getrocknet werden, zum Erhalt von magensaftresistent befilmten NSAID-Pellets, und Mischen der magensaftresistent befilmten NSAID-Pellets mit den PPI-Pellets, wobei die Rührflügelgeschwindigkeit des Schnellmischers größer oder gleich 50 U/min ist, vorzugsweise größer oder gleich 65 U/min, noch bevorzugter im Bereich von 70 bis 140 U/min liegt, die Granulierzeit vorzugsweise kleiner als 20 Minuten, noch bevorzugter kleiner oder gleich 10 Minuten, besonders bevorzugt kleiner oder gleich 8 Minuten und am bevorzugtesten 5 bis 8 Minuten ist, und das Granulieren vorzugsweise in Abwesenheit von Wasser geschieht.

4. Verfahren gemäß Anspruch 3, wobei ein weiterer Teil des NSAID zusammen mit mindestens einem pharmazeutisch annehmbaren Hilfsstoff zu NSAID-haltigen Kernen verarbeitet und diese mit einer für das NSAID retardiert durchlässigen Diffusionsmembran umhüllt werden, zum Erhalt retardiert freisetzender NSAID-Pellets, wobei die retardiert freisetzenden NSAID-Pellets gegebenenfalls auf eine Restfeuchte von kleiner oder gleich 2 Gew.-%, vorzugsweise von kleiner oder gleich 1,5 Gew.-%, besonders bevorzugt von kleiner oder gleich 1,0 Gew.-% getrocknet werden, und Vermischen der retardiert freisetzenden NSAID-Pellets mit den PPI-Pellets und gegebenenfalls den magensaftresistent befilmten NSAID-Pellets.

5. Verfahren gemäß Anspruch 3 oder 4, wobei das NSAID ausgewählt ist aus Diclofenac, Ibuprofen, Ketoprofen, Naproxen, Indometacin, Piroxicam, Meloxicam, Acetylsalicylsäure, Celecoxib; Parecoxib und Etoricoxib, vorzugsweise aus Diclofenac.

6. Verfahren gemäß Anspruch 4 oder 5, wobei das Stoffmengenverhältnis von NSAID in magensaftresistent befilmten Pellets zu NSAID in retardiert freisetzenden Pellets 0,1:1 bis 1:1, vorzugsweise 0,5:1 bis 1:1 beträgt.

7. Verfahren gemäß einem der Ansprüche 1 bis 6, wobei die PPI-Pellets nach der Herstellung einen Wassergehalt von kleiner oder gleich 1,0 Gew.-%, vorzugsweise von kleiner oder gleich 0,5 Gew.-% aufweisen.

8. Verfahren gemäß einem der Ansprüche 1 bis 7, wobei die mindestens eine basische Komponente unabhängig ausgewählt ist aus der Gruppe bestehend aus: Magnesiumcarbonat, Magnesiumoxid, Magnesiumhydroxid, Aluminiumcarbonat, Aluminiumhydroxid, Calciumcarbonat, Calciumphosphat, Calciumcitrat, Natriumcarbonat, Natriumhydrogencarbonat, Natriumphosphat, Natriumcitrat, Kaliumcarbonat, Kaliumphosphat und Kaliumcitrat, vorzugsweise Magnesiumcarbonat.

9. Verfahren gemäß einem der Ansprüche 1 bis 8, wobei der mindestens eine Hilfsstoff unabhängig ausgewählt ist aus der Gruppe bestehend aus: Mannitol, Sorbitol, Isomalt, hochdisperses Siliziumdioxid, Mikrokristalline Cellulose, Dextrin, Maltodextrin, Maisstärke, Saccharose, Lactose und Natriumlaurylsulfat, vorzugsweise aus Mannitol und Natriumlaurylsulfat.

10. Verfahren gemäß einem der Ansprüche 1 bis 9, wobei die inerte Zwischenschicht umfasst oder unabhängig ausgewählt ist aus Hydroxypropylcellulose, Hydroxypropylmethylcellulose, Polyvinylpyrrolidon, Polyvinylalkohol und Mischungen davon, insbesondere Hydroxypropylmethylcellulose.

11. Verfahren gemäß einem der Ansprüche 1 bis 10, wobei die magensaftresistente Schicht unabhängig ausgewählt ist aus: Methacrylsäure-Ethylacrylat-Copolymer, Methacrylsäure-Methylmethacrylat-Copolymer, Polyvinylacetatphthalat, Hydroxypropylmethylcelluloseacetatphthalat, Celluloseacetatphthalat, und Hydroxypropylmethylcelluloseacetatsuccinat, insbesondere Methacrylsäure-Ethylacrylat-Copolymer.

12. Pharmazeutische Zubereitung erhältlich durch ein Verfahren gemäß einem der Ansprüche 1 bis 11, wobei die Pellets vorzugsweise in einer Gelatinekapsel, insbesondere einer Hartgelatinekapsel eingeschlossen sind.

13. Pharmazeutische Zubereitung gemäß Anspruch 12, wobei die magensaftresistent beschichteten Pellets eine Auflösungsgeschwindigkeit besitzen, die beim Messen *in vitro* in einer Drehkörbchen- bzw. Blattrührerapparatur nach dem Europäischen Arzneibuch Version 6 in Phosphatpuffer bei einer Lösungsmittelmenge von 900 ml bei 100 U/min und 37 °C im wesentlichen dem folgenden Auflösungsmuster entspricht:
(i) nach 2 h Messzeit bei pH 1,2 sind 10 % oder weniger, vorzugsweise 5 % oder weniger des gesamten PPI gelöst, und
(ii) nach 2 h Messzeit bei pH 1,2 und nach 10 min Messzeit bei pH 6,8 sind 80 % oder mehr, vorzugsweise 90 % oder mehr des gesamten PPI gelöst.

14. Pharmazeutische Zubereitung gemäß Anspruch 12 oder 13, wobei die magensaftresistent befilmten Pellets eine Auflösungsgeschwindigkeit besitzen, die beim Messen *in vitro* in einer Drehkörbchen- bzw. Blattrührerapparatur nach dem Europäischen Arzneibuch Version 6 in Salzsäure bzw. Phosphatpuffer bei einer Lösungsmittelmenge von 900 ml bei 100 U/min und 37 °C im wesentlichen dem folgenden Auflösungsmuster entspricht:
(i) nach 2 h Messzeit bei pH 1,2 sind 10 % oder weniger, vorzugsweise 5 % oder weniger des NSAID gelöst, und
(ii)nach 1 h Messzeit bei pH 6,8 sind 80 % oder mehr des NSAID gelöst.

15. Pharmazeutische Zubereitung gemäß Anspruch 12, 13 oder 14, die beim Messen *in vitro* in einer Drehkörbchen- bzw. Blattrührerapparatur nach dem Europäischen Arzneibuch Version 6 in Salzsäure bzw. Phosphatpuffer bei einer Lösungsmittelmenge von 900 ml bei 100 U/min und 37 °C die im wesentlichen folgende Auflösungsgeschwindigkeit aufweist:
(i) nach 2 h Messzeit bei pH 1,2 sind 10 % oder weniger, vorzugsweise 5 % oder weniger des gesamten PPI und 10 % oder weniger, vorzugsweise 5 % oder weniger des gesamten NSAID gelöst,
(ii) nach 10 min Messzeit bei pH 6,8 sind 80% oder mehr, vorzugsweise 90 % oder mehr des gesamten PPI gelöst, und
(iii) nach 1 h Messzeit bei pH 6,8 sind 40 bis 70 % des gesamten NSAID gelöst, und über einen Zeitraum von weiteren 5 h Messzeit bei pH 6,8 werden weitere 2,5 bis 10 % des gesamten NSAID pro h gelöst.

16. Pharmazeutische Zubereitung gemäß Anspruch 12, 13, 14 oder 15, wobei die mindestens eine basische Komponente unabhängig ausgewählt ist aus der Gruppe bestehend aus: Magnesiumcarbonat, Magnesiumoxid, Magnesiumhydroxid, Calciumcarbonat, Calciumphosphat und Calciumcitrat, vorzugsweise Magnesiumcarbonat.

17. Pharmazeutische Zubereitung gemäß Anspruch 12, 13, 14, 15 oder 16, zur Verwendung zur Behandlung von Schmerzen und Entzündungen, insbesondere bei Rheuma, Prellungen, Zerrungen und Arthrose, vorzugsweise bei Rheuma.

## Claims

1. Method of producing a pharmaceutical preparation that contains a PPI (proton pump inhibitor) in the form of spherical granules (pellets), comprising the following steps:
(a) granulating the PPI, at least one basic component, at least one pharmaceutically acceptable excipient, and at least one alcohol, preferably ethanol and/or isopropanol, in particular isopropanol, using a high-speed mixer, to obtain PPI-containing cores,
(b) drying the cores,
(c) coating the dried cores with an inert intermediate layer,
(d) film-coating the coated cores with an enteric layer, and
(e) drying the enteric film-coated cores to a residual moisture of less than or equal to 1.5 wt.-%, preferably less than or equal to 1 wt.%, to obtain PPI pellets,
wherein the impeller blade speed of the high-speed mixer is greater than or equal to 50 rev/min, is preferably greater than or equal to 65 rev/min, more preferably is in the range from 70 to 140 rev/min, the granulation time is preferably less than 20 minutes, more preferably less than or equal to 10 minutes, particularly preferably less than or equal to 8 minutes and most preferably 5 to 8 minutes, and the granulating (a) takes place in the absence of water.

2. Method according to claim 1, wherein the PPI is selected from omeprazole, esomeprazole, lansoprazole, pantoprazole and rabeprazole, preferably omeprazole.

3. Method according to claim 1 or 2, wherein the pharmaceutical preparation further contains an NSAID (non-steroidal anti-inflammatory drug) in the form of pellets, wherein a proportion of the NSAID
(i) in the form of a salt, for example as sodium salt, together with the PPI, is granulated, dried, coated, film-coated and dried again according to steps (a) to (e), and/or
(ii) in free form or in the form of a salt, for example as sodium salt, separately from the PPI, is granulated together with at least one pharmaceutically acceptable excipient and at least one alcohol, preferably ethanol and/or isopropanol, in particular isopropanol, using a high-speed mixer, to obtain NSAID-containing cores, the cores are dried, film-coated with an enteric layer, and the enteric film-coated cores are dried to a residual moisture of less than or equal to 4.0 wt.-%, preferably of less than or equal to 3.0 wt.%, particularly preferably of less than or equal to 2.5 wt.-%, to obtain enteric film-coated NSAID pellets, and mixing the enteric film-coated NSAID pellets with the PPI pellets, wherein the impeller blade speed of the high-speed mixer is greater than or equal to 50 rev/min, preferably greater than or equal to 65 rev/min, more preferably is in the range from 70 to 140 rev/min, the granulation time is preferably less than 20 minutes, more preferably less than or equal to 10 minutes, particularly preferably less than or equal to 8 minutes and most preferably 5 to 8 minutes, and the granulation preferably takes place in the absence of water.

4. Method according to claim 3, wherein another proportion of the NSAID is processed together with at least one pharmaceutically acceptable excipient into NSAID-containing cores and these are coated with a diffusion membrane with delayed permeability for the NSAID, to obtain delayed-release NSAID pellets, wherein the delayed-release NSAID pellets optionally are dried to a residual moisture of less than or equal to 2 wt.-%, preferably of less than or equal to 1.5 wt.-%, particularly preferably of less than or equal to 1.0 wt.-%, and mixing the delayed-release NSAID pellets with the PPI pellets and optionally the enteric film-coated NSAID pellets.

5. Method according to claim 3 or 4, wherein the NSAID is selected from diclofenac, ibuprofen, ketoprofen, naproxen, indometacin, piroxicam, meloxicam, acetylsalicylic acid, celecoxib, parecoxib and etoricoxib, preferably diclofenac.

6. Method according to claim 4 or 5, wherein the molar ratio of NSAID in enteric film-coated pellets to NSAID in delayed-release pellets is 0.1:1 to 1:1, preferably 0.5:1 to 1:1.

7. Method according to one of claims 1 to 6, wherein the PPI pellets have, after production, a water content of less than or equal to 1.0 wt.-%, preferably of less than or equal to 0.5 wt.-%.

8. Method according to one of claims 1 to 7, wherein the at least one basic component is selected independently from the group consisting of: magnesium carbonate, magnesium oxide, magnesium hydroxide, aluminium carbonate, aluminium hydroxide, calcium carbonate, calcium phosphate, calcium citrate, sodium carbonate, sodium hydrogen carbonate, sodium phosphate, sodium citrate, potassium carbonate, potassium phosphate and potassium citrate, preferably magnesium carbonate.

9. Method according to one of claims 1 to 8, wherein the at least one excipient is selected independently from the group consisting of: mannitol, sorbitol, isomalt, colloidal silica, microcrystalline cellulose, dextrin, maltodextrin, maize starch, sucrose, lactose and sodium lauryl sulphate, preferably from mannitol and sodium lauryl sulphate.

10. Method according to one of claims 1 to 9, wherein the inert intermediate layer comprises or is selected independently from hydroxypropylcellulose, hydroxypropylmethylcellulose, polyvinylpyrrolidone, polyvinyl alcohol and mixtures thereof, in particular hydroxypropylmethylcellulose.

11. Method according to one of claims 1 to 10, wherein the enteric layer is selected independently from: methacrylic acid-ethylacrylate copolymer, methacrylic acid-methylmethacrylate copolymer, polyvinylacetate phthalate, hydroxypropyl methylcellulose acetate phthalate, cellulose acetate phthalate, and hydroxypropyl methylcellulose acetate succinate, in particular methacrylic acid-ethylacrylate copolymer.

12. Pharmaceutical preparation obtainable by a method according to one of claims 1 to 11, wherein the pellets are preferably enclosed in a gelatin capsule, in particular a hard gelatin capsule.

13. Pharmaceutical preparation according to claim 12, wherein the enteric-coated pellets have a dissolution rate which, when measured *in vitro* in a basket apparatus or paddle apparatus according to the European Pharmacopoeia Version 6 in phosphate buffer with an amount of solvent of 900 ml at 100 rev/min and 37°C, essentially corresponds to the following dissolution profile:
(i) after a measuring time of 2 h at pH 1.2, 10% or less, preferably 5% or less of the total PPI has dissolved, and
(ii) after a measuring time of 2 h at pH 1.2 and after a measuring time of 10 min at pH 6.8, 80% or more, preferably 90% or more of the total PPI has dissolved.

14. Pharmaceutical preparation according to claim 12 or 13, wherein the enteric film-coated pellets have a dissolution rate which, when measured *in vitro* in a basket apparatus or paddle apparatus according to the European Pharmacopoeia Version 6 in hydrochloric acid or phosphate buffer with an amount of solvent of 900 ml at 100 rev/min and 37°C, essentially corresponds to the following dissolution profile:
(i) after a measuring time of 2 h at pH 1.2, 10% or less, preferably 5% or less of the NSAID has dissolved, and
(ii) after a measuring time of 1 h at pH 6.8, 80% or more of the NSAID has dissolved.

15. Pharmaceutical preparation according to claim 12, 13 or 14, which, when measured *in vitro* in a basket apparatus or paddle apparatus according to the European Pharmacopoeia Version 6 in hydrochloric acid or phosphate buffer with an amount of solvent of 900 ml at 100 rev/min and 37°C, essentially has the following dissolution rate:
(i) after a measuring time of 2 h at pH 1.2, 10% or less, preferably 5% or less of the total PPI and 10% or less, preferably 5% or less of the total NSAID has dissolved,
(ii) after a measuring time of 10 min at pH 6.8, 80% or more, preferably 90% or more of the total PPI has dissolved, and
(iii) after a measuring time of 1 h at pH 6.8, 40 to 70% of the total NSAID has dissolved, and over a period of a further 5 h measuring time at pH 6.8, a further 2.5 to 10% of the total NSAID per h has dissolved.

16. Pharmaceutical preparation according to claim 12, 13, 14 or 15, wherein the at least one basic component is selected independently from the group consisting of: magnesium carbonate, magnesium oxide, magnesium hydroxide, calcium carbonate, calcium phosphate and calcium citrate, preferably magnesium carbonate.

17. Pharmaceutical preparation according to claim 12, 13, 14, 15 or 16, for use in treating pains and inflammations, in particular in rheumatism, contusions, sprains and arthrosis, preferably in rheumatism.

## Revendications

1. Procédé de production d'une préparation pharmaceutique contenant un IPP (inhibiteur de la pompe à protons) sous la forme de granules sphériques (pastilles), qui comprend les étapes suivantes :
(a) granulation de l'IPP, d'au moins un composant basique, d'au moins un excipient pharmaceutiquement acceptable et d'au moins un alcool, préférablement l'éthanol et/ou l'isopropanol et en particulier l'isopropanol, au moyen d'un mélangeur à grande vitesse pour obtenir des noyaux contenant l'IPP,
(b) séchage des noyaux,
(c) enrobage des noyaux séchés avec une couche intermédiaire inerte,
(d) pelliculage des noyaux enrobés avec un film gastro-résistant, et
(e) séchage des noyaux à pelliculage gastro-résistant jusqu'à ce que l'humidité résiduelle soit inférieure ou égale à 1,5 % en poids, préférablement inférieure ou égale à 1 % en poids, pour obtenir des pastilles de l'IPP,
dans lequel la vitesse de la palette d'agitation du mélangeur à grande vitesse est supérieure ou égale à 50 tours/min, préférablement supérieure ou égale à 65 tours/min et plus préférablement dans la plage de 70 à 140 tours/min, la durée de la granulation est préférablement inférieure à 20 minutes, plus préférablement inférieure ou égale à 10 minutes, particulièrement préférablement inférieure ou égale à 8 minutes et le plus préférablement de 5 à 8 minutes et l'étape de granulation (a) a lieu en l'absence d'eau.

2. Procédé selon la revendication 1, dans lequel l'IPP est sélectionné parmi l'oméprazole, l'ésoméprazole, le lansoprazole, le pantoprazole et le rabéprazole, préférablement l'oméprazole.

3. Procédé selon la revendication 1 ou 2, dans lequel la préparation pharmaceutique contient en outre un AINS (anti-inflammatoire non stéroïdien) sous la forme de pastilles, une proportion de l'AINS étant
(i) sous la forme d'un sel, par exemple d'un sel de sodium, qui est soumis, avec l'IPP, à une granulation, à un séchage, à un enrobage, à un pelliculage et à nouveau à un séchage conformément aux étapes (a) à (e) et/ou
(ii) sous une forme libre ou sous la forme d'un sel, par exemple d'un sel de sodium, qui est soumis, séparément de l'IPP, à une granulation avec un excipient pharmaceutiquement acceptable et au moins un alcool, préférablement l'éthanol et/ou l'isopropanol et en particulier l'isopropanol, au moyen d'un mélangeur à grande vitesse pour obtenir des noyaux contenant l'AINS, les noyaux étant ensuite séchés et pelliculés avec un film gastro-résistant avant d'être à nouveau séchés jusqu'à ce que l'humidité résiduelle soit inférieure ou égale à 4,0 % en poids, préférablement inférieure ou égale à 3,0 % en poids et particulièrement préférablement inférieure ou égale à 2,5 % en poids pour obtenir des pastilles de l'AINS à pelliculage gastro-résistant qui sont ensuite mélangées avec les pastilles de l'IPP, dans lequel la vitesse de la palette d'agitation du mélangeur à grande vitesse est supérieure ou égale à 50 tours/min, préférablement supérieure ou égale à 65 tours/min et plus préférablement dans la plage de 70 à 140 tours/min, la durée de la granulation est préférablement inférieure à 20 minutes, plus préférablement inférieure ou égale à 10 minutes, particulièrement préférablement inférieure ou égale à 8 minutes et le plus préférablement de 5 à 8 minutes et l'étape de granulation (a) a lieu en l'absence d'eau.

4. Procédé selon la revendication 3, dans lequel une autre proportion de l'AINS est transformée, avec au moins un excipient pharmaceutiquement acceptable, en des noyaux contenant l'AINS qui sont ensuite enrobés avec une membrane de diffusion ayant une perméabilité retardée pour l'AINS pour obtenir des pastilles de l'AINS à libération retardée, dans lequel les pastilles de l'AINS à libération retardée sont en option séchées jusqu'à ce que l'humidité résiduelle soit inférieure ou égale à 2,0 % en poids, préférablement inférieure ou égale à 1,5 % en poids et particulièrement préférablement inférieure ou égale à 1 % en poids avant mélange des pastilles d'AINS à libération retardée avec les pastilles de l'IPP et, en option, les pastilles de l'AINS à pelliculage gastro-résistant.

5. Procédé selon la revendication 3 ou 4, dans lequel l'AIN est sélectionné parmi le diclofénac, l'ibuprofène, le kétoprofène, le naproxène, l'indométacine, le piroxicam, le méloxicam, l'acide acétylsalicylique, le célécoxib, le parécoxib et l'étoricoxib, préférablement le diclofénac.

6. Procédé selon la revendication 4 ou 5, dans lequel le rapport molaire de l'AINS contenu dans les pastilles à pelliculage gastro-résistant et de l'AINS contenu dans les pastilles à libération prolongée va de 0,1:1 à 1:1, préférablement de 0,5:1 à 1:1.

7. Procédé selon l'une des revendications 1 à 6, dans lequel les pastilles de l'IPP présentent, après production, une teneur en eau inférieure ou égale à 1,0 % en poids, préférablement inférieure ou égale à 0,5 % en poids.

8. Procédé selon l'une des revendications 1 à 7, dans lequel le au moins un composant basique est indépendamment sélectionné dans le groupe consistant en les suivants : carbonate de magnésium, oxyde de magnésium, hydroxyde de magnésium, carbonate d'aluminium, hydroxyde d'aluminium, carbonate de calcium, phosphate de calcium, citrate de calcium, carbonate de sodium, hydrogénocarbonate de sodium, phosphate de sodium, citrate de sodium, carbonate de potassium, phosphate de potassium et citrate de potassium, préférablement le carbonate de magnésium.

9. Procédé selon l'une des revendications 1 à 8, dans lequel le au moins un excipient est indépendamment sélectionné dans le groupe consistant en les suivants : mannitol, sorbitol, isomalt, silice colloïdale, cellulose microcristalline, dextrine, maltodextrine, amidon de maïs, sucrose, lactose et laurylsulfate de sodium, préférablement le mannitol et le laurylsulfate de sodium.

10. Procédé selon l'une des revendications 1 à 9, dans lequel la couche intermédiaire inerte comprend l'un des agents suivants ou est indépendamment sélectionnée parmi ceux-ci : hydroxypropylcellulose, hydroxypropylméthylcellulose, polyvinylpyrrolidone, alcool polyvinylique et mélanges de ceux-ci, en particulier l'hydroxypropylméthylcellulose.

11. Procédé selon l'une des revendications 1 à 10, dans lequel le film gastro-résistant est indépendamment sélectionné parmi les suivants : copolymère acide méthacrylique-acrylate d'éthyle, copolymère acide méthacrylique-méthacrylate de méthyle, phtalate-acétate de polyvinyle, phtalate-acétate d'hydroxypropylméthylcellulose, phtalate-acétate de cellulose et succinate-acétate d'hydroxypropylméthylcellulose, en particulier un copolymère acide méthacrylique-acrylate d'éthyle.

12. Préparation pharmaceutique pouvant être obtenue par un procédé selon l'une des revendications 1 à 11, dans laquelle les pastilles sont préférablement renfermées dans une capsule de gélatine, en particulier une capsule de gélatine dure.

13. Préparation pharmaceutique selon la revendication 12, dans laquelle les pastilles à pelliculage gastro-résistant ont une vitesse de dissolution qui, quand mesurée *in vitro* dans un appareil à panier ou un appareil à palette conforme à celui décrit dans la version 6 de la Pharmacopée européenne dans un tampon phosphate en présence d'une quantité de solvant qui est de 900 ml à 100 tours/min et à 37 °C, produit essentiellement le profil de dissolution suivant :
(i) après une période de mesure de 2 h à pH 1,2, une dissolution de 10 % ou moins, préférablement de 5 % ou moins, de l'IPP total est rapportée, et
(ii) après une période de mesure de 2 h à pH 1,2 et après une période de mesure de 10 minutes à pH 6,8, une dissolution de 80 % ou plus, préférablement de 90 % ou plus, de l'IPP total est rapportée.

14. Préparation pharmaceutique selon la revendication 12 ou 13, dans laquelle les pastilles à pelliculage gastro-résistant ont une vitesse de dissolution qui, quand mesurée *in vitro* dans un appareil à panier ou un appareil à palette conforme à celui décrit dans la version 6 de la Pharmacopée européenne dans l'acide chlorhydrique ou dans un tampon phosphate en présence d'une quantité de solvant qui est de 900 ml à 100 tours/min et à 37 °C, produit essentiellement le profil de dissolution suivant :
(i) après une période de mesure de 2 h à pH 1,2, une dissolution de 10 % ou moins, préférablement de 5 % ou moins, de l'AINS total est rapportée, et
(ii) après une période de mesure de 1 h à pH 6,8, une dissolution de 80 % ou plus de l'AINS total est rapportée.

15. Préparation pharmaceutique selon la revendication 12, 13 ou 14, dont le profil de dissolution, mesuré *in vitro* dans un appareil à panier ou un appareil à palette conforme à celui décrit dans la version 6 de la Pharmacopée européenne dans l'acide chlorhydrique ou dans un tampon phosphate en présence d'une quantité de solvant qui est de 900 ml à 100 tours/min et à 37 °C, est essentiellement le suivant :
(i) après une période de mesure de 2 h à pH 1,2, une dissolution de 10 % ou moins, préférablement de 5 % ou moins, de l'IPP total et de 10 % ou moins, préférablement de 5 % ou moins, de l'AINS total est rapportée,
(ii) après une période de mesure de 10 min à pH 6,8, une dissolution de 80 % ou plus, préférablement de 90 % ou plus, de l'IPP total est rapportée, et
(iii) après une période de mesure de 1 h à pH 6.8, une dissolution de 40 à 70 % de l'AINS total est rapportée et, sur une période de mesure supplémentaire de 5 h à pH 6,8, une dissolution supplémentaire de 2,5 à 10 % par heure de l'AINS total est rapportée.

16. Préparation pharmaceutique selon l'une des revendications 12, 13, 14 ou 15, dans laquelle le au moins un composant basique est sélectionné indépendamment dans le groupe consistant en les suivants : carbonate de magnésium, oxyde de magnésium, hydroxyde de magnésium, carbonate de calcium, phosphate de calcium et citrate de calcium, préférablement le carbonate de magnésium.

17. Préparation pharmaceutique selon la revendication 12, 13, 14, 15 ou 16, pour une utilisation dans le traitement de douleurs et d'inflammations, en particulier d'un rhumatisme, de contusions, d'entorses et d'une arthrose, préférablement d'un rhumatisme.
